(19) Europäisches Patentamt / European Patent Office / Office européen des brevets

(11) **EP 1 417 487 B1**

(12) **EUROPEAN PATENT SPECIFICATION**

(45) Date of publication and mention of the grant of the patent: **17.10.2007 Bulletin 2007/42**

(21) Application number: **02721315.6**

(22) Date of filing: **08.03.2002**

(51) Int Cl.: ***G01N 33/53*** *(2006.01)* ***G01N 33/569*** *(2006.01)* ***G01N 33/542*** *(2006.01)*

(86) International application number: **PCT/US2002/007121**

(87) International publication number: **WO 2002/072606 (19.09.2002 Gazette 2002/38)**

(54) **EPITOPE TESTING USING HLA**

EPITOP-UNTERSUCHUNG MIT HLA

TESTS D'EPITOPES FAISANT APPEL AU SYSTEME HLA

(84) Designated Contracting States: **AT BE CH CY DE DK ES FI FR GB GR IE IT LI LU MC NL PT SE TR**

(30) Priority: **09.03.2001 US 274605 P**
**10.10.2001 US 974366**
**18.12.2001 US 22066**
**07.03.2002 US 362799 P**

(43) Date of publication of application: **12.05.2004 Bulletin 2004/20**

(73) Proprietor: **THE BOARD OF REGENTS OF THE UNIVERSITY OF OKLAHOMA**
**Norman,**
**Oklahoma 73109 (US)**

(72) Inventors:
- **HILDEBRAND, William, H.**
  **Edmond, OK 73034 (US)**
- **BUCHLI, Rico**
  **Edmond, OK 73034-4883 (US)**
- **PRILLIMAN, Kiley, R.**
  **G1, San Diego, CA 92126 (US)**
- **HICKMAN, Heather, D.**
  **Oklahama City, OK 73118 (US)**

(74) Representative: **Ebner von Eschenbach, Jennifer et al**
**LADAS & PARRY LLP**
**Dachauerstrasse 37**
**80335 München (DE)**

(56) References cited:
- **OJCIUS D M ET AL: "DISSOCIATION OF THE PEPTIDE-MHC CLASS I COMPLEX LIMITS THE BINDING RATE OF EXOGENOUS PEPTIDE" JOURNAL OF IMMUNOLOGY, THE WILLIAMS AND WILKINS CO. BALTIMORE, US, vol. 151, no. 11, 1 December 1993 (1993-12-01), pages 6020-6026, XP000872960 ISSN: 0022-1767**
- **OJCIUS DAVID M ET AL: "Dissociation of the peptide/MHC class I complex: pH dependence and effect of endogenous peptides on the activation energy" BIOCHEMICAL AND BIOPHYSICAL RESEARCH COMMUNICATIONS, vol. 197, no. 3, 1993, pages 1216-1222, XP002318878 ISSN: 0006-291X**
- **SETTE A ET AL: "PEPTIDE BINDING TO THE MOST FREQUENT HLA-A CLASS I ALLELES MEASURED BY QUANTITATIVE MOLECULAR BINDING ASSAYS" MOLECULAR IMMUNOLOGY, ELMSFORD, NY, US, vol. 31, no. 11, 1994, pages 813-822, XP000603774 ISSN: 0161-5890**
- **KAST W M ET AL: "ROLE OF HLA-A MOTIFS IN IDENTIFICATION OF POTENTIAL CTL EPITOPES INHUMAN PAPILLOMAVIRUS TYPE 16 E6 AND E7 PROTEINS" JOURNAL OF IMMUNOLOGY, THE WILLIAMS AND WILKINS CO. BALTIMORE, US, vol. 152, no. 8, 1994, pages 3904-3912, XP002936659 ISSN: 0022-1767**
- **DE KROON ANTON I P M: "The accessibility of peptides bound to the mouse MHC class II molecule IE-d studied by fluorescence" FEBS (FEDERATION OF EUROPEAN BIOCHEMICAL SOCIETIES) LETTERS, vol. 342, no. 3, 1994, pages 230-234, XP002318895 ISSN: 0014-5793**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 417 487 B1

- **DEDIER S ET AL: "Use of fluorescence polarization to monitor MHC-peptide interactions in solution" JOURNAL OF IMMUNOLOGICAL METHODS, ELSEVIER SCIENCE PUBLISHERS B.V.,AMSTERDAM, NL, vol. 255, no. 1-2, 1 September 2001 (2001-09-01), pages 57-66, XP004274821 ISSN: 0022-1759**
- **VAN DER BURG ET AL.: 'An HLA class I peptide-binding assay based on competition for binding to class I molecules on intact human B cells identification of conserved HIV-1 polymerase peptides binding to HLA-A*0301' HUMAN IMMUNOLOGY vol. 44, 1995, pages 189 - 198, XP000673956**
- **STEVENS ET AL.: 'Efficient generation of major histocompatibility complex class I-peptide complexes using synthetic peptide libraries' JOURNAL OF BIOLOGICAL CHEMISTRY vol. 273, no. 5, 30 January 1998, pages 2874 - 2884, XP002136475**
- **KUBY, J: "IMMUNOLOGY" 1994, FREEMAN AND CO * page 214 - page 215 ***

**Description**

BACKGROUND OF THE INVENTION

1. Field of the Invention

The present invention relates generally to a methodology of epitope testing for the identification of peptides that bind to an individual soluble MHC Class I or Class II molecule.

2. Description of the Background Art

Class I major histocompatibility complex (MHC) molecules, designated HLA class I in humans, bind and display peptide antigen ligands upon the cell surface. The peptide antigen ligands presented by the class I MHC molecule are derived from either normal endogenous proteins ("self") or foreign proteins ("nonself") introduced into the cell. Nonself proteins may be products of malignant transformation or intracellular pathogens such as viruses. In this manner, class I MHC molecules convey information regarding the internal fitness of a cell to immune effector cells including but not limited to, $CD8^+$ cytotoxic T lymphocytes (CTLs), which are activated upon interaction with "nonself" peptides, thereby lysing or killing the cell presenting such "nonself" peptides.

Class II MHC molecules, designated HLA class II in humans, also bind and display peptide antigen ligands upon the cell surface. Unlike class I MHC molecules which are expressed on virtually all nucleated cells, class II MHC molecules are normally confined to specialized cells, such as B lymphocytes, macrophages, dendritic cells, and other antigen presenting cells which take up foreign antigens from the extracellular fluid via an endocytic pathway. The peptides they bind and present are derived from extracellular foreign antigens, such as products of bacteria that multiply outside of cells, wherein such products include protein toxins secreted by the bacteria that often times have deleterious and even lethal effects on the host (e.g. human). In this manner, class II molecules convey information regarding the fitness of the extracellular space in the vicinity of the cell displaying the class II molecule to immune effector cells, including but not limited to, $CD4^+$ helper T cells, thereby helping to eliminate such pathogens the examination of such pathogens is accomplished by both helping B cells make antibodies against microbes, as well as toxins produced by such microbes, and by activating macrophages to destroy ingested microbes.

Class I and class II HLA molecules exhibit extensive polymorphism generated by systematic recombinatorial and point mutation events; as such, hundreds of different HLA types exist throughout the world's population, resulting in a large immunological diversity. Such extensive HLA diversity throughout the population results in tissue or organ transplant rejection between individuals as well as differing susceptibilities and/or resistances to infectious diseases. HLA molecules also contribute significantly to autoimmunity and cancer. Because HLA molecules mediate most, if not all, adaptive immune responses, large quantities of pure isolated HLA proteins are required in order to effectively study transplantation, autoimmunity disorders, and for vaccine development.

There are several applications in which purified, individual class I and class II MHC proteins are highly useful. Such applications include using MHC-peptide multimers as immunodiagnostic reagents for disease resistance/autoimmunity; assessing the binding of potentially therapeutic peptides; elution of peptides from MHC molecules to identify vaccine candidates; screening transplant patients for preformed MHC specific antibodies; and removal of anti-HLA antibodies from a patient. Since every individual has differing MHC molecules, the testing of numerous individual MHC molecules is a prerequisite for understanding the differences in disease susceptibility between individuals. Therefore, purified MHC molecules representative of the hundreds of different HLA types existing throughout the world's population are highly desirable for unraveling disease susceptibilities and resistances, as well as for designing therapeutics such as vaccines.

Class I HLA molecules alert the immune response to disorders within host cells. Peptides, which are derived from viral- and tumor-specific proteins within the cell, are loaded into the class I molecule's antigen binding groove in the endoplasmic reticulum of the cell and subsequently carried to the cell surface. Once the class I HLA molecule and its loaded peptide ligand are on the cell surface, the class I molecule and its peptide ligand are accessible to cytotoxic T lymphocytes (CTL). CTL survey the peptides presented by the class I molecule and destroy those cells harboring ligands derived from infectious or neoplastic agents within that cell.

While specific CTL targets have been identified, little is known about the breadth and nature of ligands presented on the surface of a diseased cell. From a basic science perspective, many outstanding questions have permeated through the art regarding peptide exhibition. For instance, it has been demonstrated that a virus can preferentially block expression of HLA class I molecules from a given locus while leaving expression at other loci intact. Similarly, there are numerous reports of cancerous cells that fall to express dass I HLA at particular loci. However, there is no data describing how (or if) the three classical HLA class I loci differ in the Immunoregulatory ligands they bind. It is therefore unclear how class I molecules from the different loci vary in their interaction with viral- and tumor-derived ligands and the number of peptides each will present.

Discerning virus- and tumor-specific ligands for CTL recognition is an important component of vaccine design. Ligands unique to tumorigenic or infected cells can be tested and incorporated into vaccines designed to evoke a protective CTL response. Several methodologies are currently employed to identify potentially protective peptide ligands. One approach uses T cell lines or clones to screen for biologically active ligands among chromatographic fractions of eluted peptides (Cox et al., Science, vol 264,1994, pages 716-719). This approach has been employed to identify peptides ligands specific to cancerous cells. A second technique utilizes predictive algorithms to identify peptides capable of binding to a particular class I molecule based upon previously determined motif and/or individual ligand sequences (De Groot et al., Emerging Infectious Diseases, (7) 4, 2001). Peptides having high predicted probability of binding from a pathogen of interest can then be synthesized and tested for T cell reactivity in precursor, tetramer or ELISpot assays.

However, there has been no readily available source of individual HLA molecules. The quantities of HLA protein available have been small and typically consist of a mixture of different HLA molecules. Production of HLA molecules traditionally involves growth and lysis of cells expressing multiple HLA molecules. Ninety percent of the population is heterozygous at each of the HLA loci; codominant expression results in multiple HLA proteins expressed at each HLA locus. To purify native class I or class II molecules from mammalian cells requires time-consuming and cumbersome purification methods, and since each cell typically expresses multiple surface-bound HLA class I or class II molecules, HLA purification results in a mixture of many different HLA class I or class II molecules. When performing experiments using such a mixture of HLA molecules or performing experiments using a cell having multiple surface-bound HLA molecules, interpretation of results cannot *directly* distinguish between the different HLA molecules, and one cannot be certain that any particular HLA molecule is responsible for a given result. Therefore, prior to the present invention, a need existed in the art for a method of producing substantial quantities of individual HLA class I or class II molecules so that they can be readily purified and isolated independent of other HLA class I or class II molecules. Such individual HLA molecules, when provided in sufficient quantity and purity as described herein, provides a powerful tool for studying and measuring immune responses.

Therefore, there exists a need in the art for improved methods of assaying binding of peptides to class I and class II MHC molecules to identify epitopes that bind to specific individual class I and class II MHC molecules. The present invention solves this need by coupling the production of soluble HLA molecules with epitope Isolation, discovery, and testing methodology.

In Ojcius, D.M., et al., Journal of Immunology, 151(11): 6020-6026 (1993), the authors analyzed peptide binding to recombinant single-chain MHC class I molecule dimers by determining the influence of excess competitor peptide on the dissociation of the peptide-class I complex, and by measuring binding of different peptide concentrations to recombinant single-chain class I molecule dimers already containing low affinity or high affinity peptides. Single-chain class I MHC heterodimers consisting of a heavy chain and light chain were produced in yeast or lepidopteran cell lines, purified and thereafter, incubated *in vitro* with a synthesized peptide to form the peptide-class I complex.

In Ojcius, D.M., et al., Biochemical and Biophysical Research Communications, 197(3): 1216-1222 (1993), similar studies utilizing recombinant single-chain MHC class I molecule dimers are reported in which pH dependence and effect of endogenous peptides on the activation energy of the dissociation of the peptide-MHC class I complex are reviewed.

## BRIEF DESCRIPTION OF THE DRAWINGS

FIG. 1. Overview of 2 stage PCR strategy to amplify a truncated version of the human class I MHC.

FIG. 2. Flow chart of the epitope discovery of C-terminal-tagged sHLA molecules. Class I positive transfectants are Infected with a pathogen of choice and sHLA preferentially purified utilizing the tag. Subtractive comparison of MS ion maps yields ions present only in infected cell, which are then MS/MS sequenced to derive class I epitopes.

FIG. 3. A*0201T saturation curve of increasing amount of peptide/sHLA complex loaded on a coated W6/32 ELISA plate.

FIG. 4. Comparison of saturation curves created using either sHLA allele (A*0201T) or detergent lysate A*0201 obtained through cell extraction demonstrating higher sensitivity of sHLA than detergent lysate.

FIG. 5. Diagrammatic scheme of the peptide exchange reaction using Fluorescence Polarization.

FIG. 6. Binding of P2 to sHLA at room temperature showing net increase after t =0 subtraction.

FIG. 7. Binding of P2 to sHLA at 4°C (preincubated at 54°C) showing net increase after t=0 subtraction.

FIG. 8. Binding of various peptides (P1- P5) to sHLA A*0201T over a range of concentration detected by Fluorescence Polarization at time 45 hours after mixing.

FIG. 9. Binding of various peptides (P1 - P5) to sHLA A*0201T using Fluorescence Polarization at a concentration of 50 μg/ml sHLA.

FIG. 10. Competition assay for sHLA A*0201T loaded with the specific standard peptide P5(FITC).

DETAILED DESCRIPTION OF THE INVENTION

The present invention combines methodologies for assaying the binding of peptide epitopes to individual, soluble MHC molecules with methodologies for the production of Individual, soluble MHC molecules and with a method of epitope discovery and comparative ligand mapping (including methods of distinguishing Infected/tumor cells from uninfected/non-tumor cells).

The present invention is related to a method of assaying a peptide for binding to an individual class I molecule. The present invention is directed to a method of assaying a peptide for binding to an individual soluble MHC class I molecule as recited in claim 1 hereof. The method includes providing a peptide of interest and providing individual soluble class I molecule-endogenous peptide complexes in which Individual soluble class I molecules have endogenous peptides loaded therein. The peptide of interest and the individual soluble class I molecules are mixed together, and Individual soluble class I molecule-peptide of interest complexes, wherein the individual soluble class I molecules have the peptide of Interest loaded therein, are Identified. The Individual soluble class I molecule-endogenous peptide complexes may be treated under conditions that cause the individual soluble class I molecules to release the endogenous peptides prior to mixing the peptide of interest with the individual soluble class I molecules, and such method of treating may involve heating the individual soluble class I molecule-endogenous peptide complexes to cause the individual soluble class I molecules to release the endogenous peptides.

The peptide of interest may be labeled using a radiolabel or a fluorescent label to allow identification of individual soluble class I molecule-peptide, of interest complexes from unbound peptide of interest. When a fluorescent label is utilized, the individual soluble class I molecule-peptide of interest complexes may be identified by any of the methods of fluorescence detection known in the art, such as by fluorescence polarization. Alternatively, the individual soluble class I molecule-fluorescence labeled peptide of interest may be isolated using an antibody against the individual soluble class I molecule. When a radiolabel is utilized, the individual soluble class I molecule-peptide of interest complexes may be isolated away from unbound peptide of interest, such as by gel filtration. Alternatively, the individual soluble class I molecule-peptide of interest complexes may be identified using an antibody that recognizes the individual soluble class I molecule having a peptide loaded therein.

To produce the individual soluble class I molecule-endogenous peptide complexes, genomic DNA or cDNA encoding at least one class I molecule is obtained, and an allele encoding an individual class I molecule in the genomic DNA or cDNA is identified. The allele encoding the individual class I molecule is PCR amplified in a locus specific manner such that a PCR product produced therefrom encodes a truncated, soluble form of the individual class I molecule. The PCR product is then cloned into an expression vector, thereby forming a construct that encodes the individual soluble class I molecule, and the construct is transfected into a cell line to provide a cell line containing a construct that encodes an individual soluble class I molecule. The cell line must be able to naturally process proteins into peptide ligands capable of being loaded into antigen binding grooves-of class I molecules.

The cell line is then cultured under conditions which allow for expression of the individual soluble class I molecules from the construct, and these conditions also allow for endogenous loading of a peptide ligand into the antigen binding groove of each individual soluble class I molecule prior to secretion of the individual soluble class I molecules from the cell. The secreted Individual soluble class I molecules having the endogenously loaded peptide ligands bound thereto are then isolated.

The construct that encodes the individual soluble class I molecule may further encode a tag, such as a HIS tail or a FLAG tail, which is attached to the individual soluble class I molecule and aids in isolating the individual soluble class I molecule.

The peptide of interest may be chosen based on several methods of epitope discovery known in the art. Alternatively, the peptide of interest may be identified by a method for identifying at least one endogenously loaded peptide ligand that distinguishes an infected cell from an uninfected cell. Such method includes providing an uninfected cell line containing a construct that encodes an individual soluble class I molecule, wherein the uninfected cell line is able to naturally process proteins into peptide ligands capable of being loaded into antigen binding grooves of class I molecules. A portion of the uninfected cell line is infected with at least one of a microorganism (such as HIV or HBV), a gene from a microorganism or a tumor gene, thereby providing an infected cell line, and both the uninfected cell line and the infected cell line are cultured under conditions which allow for expression of individual soluble class I molecules from the construct. The culture conditions also allow for endogenous loading of a peptide ligand in the antigen binding groove of each individual soluble class I molecule prior to secretion of the individual soluble class I molecules from the cell. The secreted individual soluble class I molecules having the endogenously loaded peptide ligands bound thereto are isolated from the uninfected cell line and the infected cell line, and the endogenously loaded peptide ligands are separated from the individual soluble class I molecules from both the uninfected cell line and the infected cell line. The endogenously loaded peptide ligands are then isolated from both the uninfected cell line and the infected cell line, and the two sets of endogenously loaded peptide ligands are compared to identify at least one endogenously loaded peptide ligand presented by the individual soluble class I molecule on the infected cell line that is not presented by the individual soluble class I molecule on the uninfected cell line, or to identify at least one endogenously loaded peptide ligand presented by the individual soluble class I molecule on the uninfected cell line that is not presented by the individual soluble class I molecule on the infected cell line.

Following identification of the peptide ligand that distinguishes an infected cell from an uninfected cell, a source protein from which the endogenously loaded peptide ligand is obtained can be identified. Such source protein may be encoded by at least one of the microorganism, the gene from a microorganism or the tumor gene with which the cell line was infected to form the infected cell line, or the source protein may be encoded by the uninfected cell line. When the source protein is encoded by the uninfected cell line, such protein may also demonstrate increased expression in a tumor cell line.

**Production of Individual, Soluble MHC Molecules**

The methods of the present invention may, in one embodiment, utilize a method of producing MHC molecules (from genomic DNA or cDNA) that are secreted from mammalian cells in a bioreactor unit. Substantial quantities of individual MHC molecules are obtained by modifying class I or class II MHC molecules so that they are capable of being secreted, isolated, and purified. Secretion of soluble MHC molecules overcomes the disadvantages and defects of the prior art in relation to the quantity and purity of MHC molecules produced. Problems of quantity are overcome because the cells producing the MHC do not need to be detergent lysed or killed in order to obtain the MHC molecule. In this way the cells producing secreted MHC remain alive and therefore continue to produce MHC. Problems of purity are overcome because the only MHC molecule secreted from the cell is the one that has specifically been constructed to be secreted. Thus, transfection of vectors encoding such secreted MHC molecules into cells which may express endogenous, surface bound MHC provides a method of obtaining a highly concentrated form of the transfected MHC molecule as it is secreted from the cells. Greater purity is assured by transfecting the secreted MHC molecule into MHC deficient cell lines,

Production of the MHC molecules In a hollow fiber bioreactor unit allows cells to be cultured at a density substantially greater than conventional liquid phase tissue culture permits. Dense culturing of cells secreting MHC molecules further amplifies the ability to continuously harvest the transfected MHC molecules. Dense bioreactor cultures of MHC secreting cell lines allow for high concentrations of individual MHC proteins to be obtained. Highly concentrated individual MHC proteins provide an advantage in that most downstream protein purification strategies perform better as the concentration of the protein to be purified increases. Thus, the culturing of MHC secreting cells in bioreactors allows for a continuous production of individual MHC proteins in a concentrated form.

The method of producing MHC molecules utilized in the present invention begins by obtaining genomic or complementary DNA which encodes the desired MHC class I or class II molecule. Alleles at the locus which encode the desired MHC molecule are PCR amplified in a locus specific manner. These locus specific PCR products may include the entire coding region of the MHC molecule or a portion thereof. In one embodiment a nested or hemi-nested PCR is applied to produce a truncated form of the class I or class II gene so that it will be secreted rather than anchored to the cell surface. FIG. 1 illustrates the PCR products resulting from such nested PCR reactions. In another embodiment the PCR will directly truncate the MHC molecule.

Locus specific PCR products are cloned into a mammalian expression vector and screened with a variety of methods to identify a clone encoding the desired MHC molecule. The cloned MHC molecules are DNA sequenced to ensure fidelity of the PCR. Faithful truncated clones of the desired MHC molecule are then transfected into a mammalian cell line. When such cell line is transfected with a vector encoding a recombinant class I molecule, such cell line may either lack endogenous class I MHC molecule expression or express endogenous class I MHC molecules. One of ordinary skill of the art would note the importance, given the present invention, that cells expressing endogenous class I MHC molecules may spontaneously release MHC into solution upon natural cell death. In cases where this small amount of spontaneously released MHC is a concern, the transfected class I MHC molecule can be "tagged" such that it can be specifically purified away from spontaneously released endogenous class I molecules in cells that express class I molecules. For example, a DNA fragment encoding a HIS tail may be attached to the protein by the PCR reaction or may be encoded by the vector into which the PCR fragment is cloned, and such HIS tail, therefore, further aids in the purification of the class I MHC molecules away from endogenous class I molecules. Tags beside a histidine tail have also been demonstrated to work, and one of ordinary skill in the art of tagging proteins for downstream purification would appreciate and know how to tag a MHC molecule in such a manner so as to increase the ease by which the MHC molecule may be purified.

Cloned genomic DNA fragments contain both exons and introns as well as other non-translated regions at the 5' and 3' termini of the gene. Following transfection into a cell line which transcribes the genomic DNA (gDNA) into RNA, cloned genomic DNA results in a protein product thereby removing introns and splicing the RNA to form messenger RNA (mRNA), which is then translated into an MHC protein. Transfection of MHC molecules encoded by gDNA therefore facilitates reisolation of the gDNA, mRNA/cDNA, and protein. Production of MHC molecules in non-mammalian cell lines such as insect and bacterial cells requires cDNA clones, as these lower cell types do not have the ability to splice introns out of RNA transcribed from a gDNA clone. In these instances the mammalian gDNA transfectants of the present invention provide a valuable source of RNA which can be reverse transcribed to form MHC cDNA. The cDNA can then be cloned, transferred into cells, and then translated into protein. In addition to producing secreted MHC, such gDNA transfectants therefore provide a ready source of mRNA, and therefore cDNA clones, which can then be transfected into non-mammalian cells for production of MHC. Thus, the present invention which starts with MHC genomic DNA clones allows for the production of MHC in cells from various species.

A key advantage of starting from gDNA is that viable cells containing the MHC molecule of interest are not needed. Since all individuals in the population have a different MHC repertoire, one would need to search more than 500,000 individuals to find someone with the same MHC complement as a desired individual - such a practical example of this principle is observed when trying to find a donor to match a recipient for bone marrow transplantation. Thus, If it is desired to produce a particular MHC molecule for use in an experiment or diagnostic, a person or cell expressing the MHC allele of interest would first need to be identified. Alternatively, in the method of the present invention, only a saliva sample, a hair root, an old freezer sample, or less than a milliliter (0.2 ml) of blood would be required to isolate the gDNA. Then, starting from gDNA, the MHC molecule of interest could be obtained via a gDNA clone as described herein, and following transfection of such clone into mammalian cells, the desired protein could be produced directly in mammalian cells or from cDNA in several species of cells using the methods of the present invention described herein.

Current experiments to obtain an MHC allele for protein expression typically start from mRNA, which requires a fresh sample of mammalian cells that *express* the MHC molecule of interest. Working from gDNA does not require gene expression or a fresh biological sample. It is also important to note that RNA is inherently unstable and is not as easily obtained as is gDNA. Therefore, if production of a particular MHC molecule starting from a cDNA clone is desired, a person or cell line that is expressing the allele of interest must traditionally first be identified in order to obtain RNA. Then a fresh sample of blood or cells must be obtained; experiments using the methodology of the present invention show that ≥5 milliliters of blood that is less than 3 days old is required to obtain sufficient RNA for MHC cDNA synthesis. Thus, by starting with gDNA, the breadth of MHC molecules that can be readily produced is expanded. This is a key factor in a system as polymorphic as the MHC system; hundreds of MHC molecules exist, and not all MHC molecules are readily available. This is especially true of MHC molecules unique to isolated populations or of MHC molecules unique to ethnic minorities. Starting class I or class II MHC molecule expression from the point of genomic DNA simplifies the isolation of the gene of interest and insures a more equitable means of producing MHC molecules for study; otherwise, one would be left to determine whose MHC molecules are chosen and not chosen for study, as well as to determine which ethnic population from which fresh samples cannot be obtained and therefore should not have their MHC molecules included in a diagnostic assay.

While cDNA may be substituted for genomic DNA as the starting material, production of cDNA for each of the desired HLA class I types will require hundreds of different, HLA typed, viable cell lines, each expressing a different HLA class I type. Alternatively, fresh samples are required from individuals with the various desired MHC types. The use of genomic DNA as the starting material allows for the production of clones for many HLA molecules from a single genomic DNA sequence, as the amplification process can be manipulated to mimic recombinatorial and gene conversion events. Several mutagenesis strategies exist whereby a given class I gDNA clone could be modified at either the level of gDNA or at the cDNA resulting from this gDNA clone, The process of producing MHC molecules utilized in the present invention does not require viable cells, and therefore the degradation which plagues RNA is not a problem.

**Method of Epitope Testing**

Utilizing the production of individual soluble MHC proteins described herein previously, a method of epitope testing has been developed. Such a method of epitope testing is advantageous because of the single specificities of the soluble HLA molecules produced in the manner described herein. Previous work in this area has used mixtures of different HLA molecules to purify or separate out individual class I or class II HLA molecules from the mixture. However, such purification steps result in selective purification of particular endogenously loaded peptide/HLA complexes while not purifying the same HLA molecule complexed with a different peptide. Such selective purification will bias the endogenously loaded peptides in the HLA molecules. For example, typical purification methods involve the use of antibodies, which may recognize certain peptides loaded in HLA molecules and not others. In addition, such purification steps may not remove all of the other class I and class II HLA molecules, leaving these proteins to skew data and its interpretation. Therefore, the antibody may not recognize all of a particular class I or class II HLA because of the peptide bound therein. Using individual, soluble HLA molecules produced in accordance with the present invention in the methods of epitope testing described herein, the individual, soluble HLA molecules are endogenously loaded with thousands of naturally produced peptides, and the pool of such individual, soluble HLA molecules is not biased in the peptide cargo loaded therein.

The goal of the epitope binding assay is to identify the affinity of peptide ligands for binding to particular HLA molecules. The initial HLA binding studies utilized detergent solubilized class I molecules from EBV transformed cell lines (Sette, A., et al., Mol Immunol, 31(11):813-22 (1994). One perceived advantage of utilizing HLA that is naturally loaded with thousands of endogenous peptide ligands inside the cell is that peptide binding competition assays actually utilize these thousands of naturally loaded peptides in the assay. In the competitive assay with naturally loaded HLA, the HLA molecule of interest can be purified away from other HLA molecules in the detergent lysate or be used in a mixture with other HLA molecules. Radiolabeled peptides are identified that have a high affinity for the HLA molecule in question. The affinity of additional "test" peptides for the HLA molecule in question is then determined by their ability to compete with the high affinity radiolabeled peptide.

Several challenges are associated with the isolation of HLA with naturally loaded peptides. One challenge is that most EBV cell lines express 6 different class I molecules. Purification of HLA molecules can result in the isolation of class I containing only a subset of the representative peptide ligands (Solheim, J.C., et al., J. Immunol., 151(10): 5387-5397 (1993); and Bluestone, J.A., et al., Journal of Exp. Med., 176(6):1757-61 (1992). Alternatively, using a mixture of HLA molecules produces results where the investigator does not know which HLA molecule in the mixture actually bound the peptide. A third challenge is that isolation of HLA proteins from detergent cell lysates produces microgram quantities of peptide. When performing a peptide binding assay twice in triplicate with hundreds to thousands of peptides at several different concentrations for each peptide, HLA protein can be rapidly utilized in milligram quantities.

To circumvent this limitation in the amount of HLA protein available with naturally loaded ligands, two groups have reported producing HLA proteins in bacteria (Ostergaard Pedersen, L., et al., Eur J Immunol, 31(10):2986-96 (2001); and Dedier, S., et al., J Immunol Methods, 255(1-2):57-66 (2001). The HLA produced in bacteria must then be refolded into a natural conformation consisting of class I heavy chain, beta-2-microglobulin light chain, and peptide ligand (s). While this method of class I protein production circumvents the limitations of detergent lysate HLA protein production, production in bacteria (or insect cells) introduces another set of challenges to obtaining HLA protein for a peptide binding study.

One challenge of producing non-native HLA heavy chains and then refolding *in vitro* is that refolding in vitro can be difficult. Discussions with Dr. John Altman at Emory University, The Beckman Tetramer Facility, and others who work with bacterial HLA Indicates that the percentage of HLA that refolds Into an intact trimolecular complex of heavy chain-light chain-peptide can range between 0 to 70%. Some HLA molecules come back together well while others do not. One factor in refolding HLA molecules *in vitro* is the peptide; high affinity peptides help to reform the complex while some peptides result in no HLA refolding. Using those that do refold with a high affinity peptide in a peptide binding assay can be difficult because displacing a single high affinity peptide can be difficult in the binding assay. This is in contrast to the various affinities of the thousands of endogenously loaded peptides. On the other hand, some HLA molecules cannot be refolded when made in bacteria. Finally, although much HLA heavy chain can be made, sometimes only a fraction of the protein will refold.

The method of producing sHLA with thousands of naturally loaded endogenous peptides as disclosed herein above allows for the production of milligram quantities of HLA In a natural form. Peptides produced in this manner have been characterized and shown to be the same as detergent solubilized HLA (Prilliman, K.R., et al, Immunogenetics, 45: 379-385 (1997); Prilliman, K.R., et al., Immunogenetics, 48:89-97 (1998); Prilliman, K.R., et al., Tissue Antigens, 54(5): 450-60 (1999); and Prilliman, K.R., et al., J Immunol, 162(12):7277-84 (1999). By secreting HLA proteins we insure that only the desired HLA molecule is in solution. Such HLA molecules can then be purified without biasing the peptides In the HLA protein. It has also been shown that high-affinity peptides known to bind a particular HLA specificity bind the HLA molecule in question, while peptides known to bind other HLA molecules do not bind the HLA molecule in question. Additionally, it has been shown that we can label the high affinity peptides with fluorescent labels and then perform a competition assay that demonstrates that other peptides can compete with the high affinity labeled peptide at increasing concentrations. We therefore have produced substantial quantities of a sHLA reagent that is natural in its conformation and peptide cargo and that is useful for peptide binding assays. The plentiful amount of protein allows the screening of hundreds or thousands of peptides at different concentrations in multiple experiments. The naturally loaded peptide provides a true competitive reflection of the affinity of the labeled and test peptides for the HLA molecule in question.

In one embodiment of the method of epitope testing, purified, individual, soluble HLA molecules containing endogenously loaded peptide ligands are mixed with a control peptide known to have high affinity for the HLA molecule and the peptide(s) of interest. A method of detection for binding of the known high affinity control peptide is provided, such as labeling of the control peptide, i.e., fluorescence or radiolabeling, or providing an antibody specific for the complex formed when the known high affinity control peptide binds to the individual HLA molecule (FIGS. 3 and 4). The detectable, high affinity control peptide will displace the endogenously loaded peptide ligands and bind to the individual soluble HLA molecule, as detected by measurement of the label on the control peptide or antibody binding to the control peptide/HLA complex, and the peptide(s) of interest will compete with the high affinity control peptides for displacement of the endogenously loaded peptides and binding to the individual soluble HLA molecules. A negative control used in such method would be a low affinity peptide which will not compete with the high affinity control peptide for displacement of the endogenously loaded peptide and binding to the individual soluble HLA molecules. Thus, epitope testing utilizing the soluble HLA molecules of the present invention, simply put, involves a competition between the control peptide and the test peptide by determining how well did the test peptide displace the control peptide from a soluble HLA molecule and/or displace an endogenously loaded peptide and competitively bind to a soluble HLA molecule.

The above-described assay will typically be performed in liquid phase; however, the method of epitope testing described herein is adaptable to solid phase assays. That is, the individual HLA molecules may be bound to a support. Methods of binding proteins to a support are known in the art and are adaptable to the assay methods described herein.

In yet another embodiment of a method of epitope testing using the individual soluble HLA molecules described herein, a number of individual soluble HLA molecules may be bound to Luminex beads. In the Luminex technology, a series of 100 to 1000 beads are each provided with varying concentrations of two different dyes. For example, Bead A may comprise 10% Dye A and 90% Dye B, while Bead B comprises 15% Dye A and 85% Dye B, while Bead C comprises 20% Dye A and 80% Dye B, and Bead D comprises 25% Dye A and 75% Dye B, etc. A specific fluorescence detector can identify each specific bead by the amounts of Dye A and Dye B present in each bead. To each of the 100 to 1000 Luminex beads, a different individual class I or II HLA molecule can be bound. It has been demonstrated that the individual soluble HLA molecules of the present invention can be bound to the Luminex beads. Therefore, the fluorescence detector can identify specific individual HLA molecules by the specific amount of dyes in the bead to which such molecules are bound. All of the beads may be mixed together in one assay, such as one well on a 96 well plate. To the reaction, a detectable, high affinity control peptide as described above is added for each individual soluble HLA molecule bound to a bead, and the peptide of interest is also added to the reaction. The mixture is then passed through the fluorescence detector, which contains two different lasers. The first laser detects the amounts of dyes in the bead, that is, the first laser identifies which bead is passing the laser. The second laser is positioned so that upon identification of the specific bead passing the first laser, the second laser can detect whether or not the detectable high affinity control peptide is bound to the HLA molecule attached to the specific bead. As stated above, this detection may be a radiolabel or fluorescence label attached to the control peptide or a method of antibody binding which detects the control peptide/HLA complex. In this manner, 100 to 1000 binding tests can be run for each peptide of interest to determine which HLA molecules bind such peptide.

In another embodiment of the method of epitope testing, as HLA molecules are known to bind 9 amino acid peptides in the groove thereof, nonamer peptides may be tethered to a chip or other type of support to provide a peptide microarray. For example, PVDF membranes have been utilized to prepare protein microarrays, and such technology could be easily adaptable to the methods described herein. Such nonamers may consist of every possible combination of amino acids, and therefore $9^{20}$ combinations of nonamers would be utilized, or such nonamers may only differ at positions known to be important for binding to HLA molecules, for example, positions 2 and 9 (in this case, $2^{20}$ combinations would be required). The nonamers may be anchored to the chip or other type of support in any known manner. Preferably, the nonamers are anchored internally off side chains of amino acids not on the ends of the nonamers. Specific individual class I or class II HLA molecules could then be passed over the nonamer microarray and allowed to bind, and by detecting binding of specific class I or class II HLA molecules to certain peptides, a database of all peptides to which individual class I or class II HLA molecules bind could be formed. In this manner, rather than making all the different combinations of peptides produced from a specific virus, bacteria, tumor gene, etc. to determine which peptides bound to individual class I or class II MHC molecules, the genomic sequence of such virus, bacteria, etc. could be "blasted" against the data obtained above and located in the database to identify putative epitopes which could be utilized in the method of epitope testing described herein.

In another embodiment of the method of epitope testing, the above described technology could be "flipped", that is, the individual HLA molecules could be bound to the chip or other support to form an HLA protein microarray. Such method would be similar to the solid-phase assays described herein before.

**Peptides Utilized in Epitope Testing**

The peptides to be tested in the epitope binding assay of the present invention will be synthetic peptides and will have their sequence derived from portions of various host, viral and bacterial proteins. The proteins upon which the peptides are based can be identified in several ways. One method of identifying proteins that contain peptides that may or may not bind to HLA molecules is through the use of T lymphocytes. T lymphocytes are known to recognize specific peptides in the context of specific HLA molecules. Indeed, this is the mechanism whereby T lymphocytes specifically target antigens in adaptive immune responses.

There are several means of using T lymphocytes for identifying HLA holding peptides that trigger the T lymphocyte. One means is to separate the complex mixture of peptides eluted from HLA molecules into fractions or points in time. The peptides in the fractions are then loaded onto HLA molecules and the cells with the loaded peptides are exposed to T lymphocytes from a cancer patient, a virus infected person, or a person with a bacterial infection. The Idea is that the T lymphocytes from a person with a disease controlled by T lymphocytes can be used to identify T lymphocyte peptide-HLA immune targets.

There are several ways to measure the recognition of HLA-peptide target cells using T lymphocytes. One method is to detect HLA/peptide target cell lysis by T lymphocytes, as disclosed in Smith, E.S., et al., Lethality-based selection of recombinant genes in mammalian cells; application to Identifying tumor antigens. Nat Med, 2001. 7(8): p. 967-72; Huczko, E.L., et al., Characteristics of endogenous peptides eluted from the class I MHC molecule HLA-B7 determined by mass spectrometry and computer modeling, J. Immunol.,1993. 151: p. 2572-2587; Scheibenbogen, C., et al., Analysis of the T cell response to tumor and viral peptide antigens by an IFNg-ELISPOT assay, Int. J. Cancer, 1997. 71: p. 932-936; and Rinaldo, C.R., Jr., et al., Anti-Human Immunodeficiency virus type 1 (HIV-1) CD8(+) T-lymphocyte reactivity during combination antiretroviral therapy In HIV-1-infected patients with advanced Immunodeficiency. J Virol, 2000. 74(9): p. 4127-38. Using one or a combination of assays that detect T lymphocyte recognition of a target cell, experiments can be designed to identify HLA-peptide candidates that may trigger T lymphocytes.

In the above references, investigators using T lymphocytes to identify HLA-peptide targets do not know which peptide is stimulating T lymphocytes. For example, Zauderer et al. know that over-expression of a transfected "tumor" gene in a cell line is causing T lymphocytes from cancer patients to recognize that cell line. However, this assay cannot determine which peptide from the protein encoded by the transfected "tumor" gene is triggering T lymphocyte recognition. Moreover, the investigator does not know if a peptide derived from the protein encoded by the "tumor" gene is presented by HLA or if the gene is indirectly changing other proteins which may indirectly result in another protein's peptide being recognized in HLA.

Other uses of T lymphocytes indicate that HLA-peptide target complexes exist, but again, they do not specify which exact target peptide is in the HLA molecule. Using evidence from the T lymphocyte assay, an investigator will synthesize several peptides which are suspected to resemble the T lymphocyte target. These peptides can then be tested for their binding to HLA target molecules. Peptides that bind to the HLA molecules are detected in this binding assay. Such binding to an HLA molecule indicates that a peptide should next be tested for recognition by T lymphocytes, Peptides that do not bind can be eliminated from further characterization.

Use of T lymphocytes is one way to begin to sift through proteins and peptide mixtures for possible immune targets. Another way is to simply synthesize all the overlapping peptides in a target protein. These overlapping peptides can be tested for their binding to HLA proteins. Peptides that bind can then be tested for T lymphocyte recognition. This process eliminates T lymphocytes from the selection process.

A protein that an investigator wishes to target in a vaccine, diagnostic, or therapy can be selected in a number of ways. These protein selection mechanisms include T lymphocyte selection, microarray identification of upregulated genes, or simply testing immune responses to a protein of interest. These selection criteria are then partnered to an epitope binding assay as described herein to identify portions of a protein that bind well to one or many HLA proteins. Another method that can be coupled to the epitope binding test described here is a predictive database. A predictive database can indicate which peptides in which proteins might bind to HLA, and then the HLA binding test described herein can confirm or deny these predictions.

The above methods for identifying epitopes for the peptide binding assay are indirect methods. That is, the peptides that may be immunogenic are not directly identified as immunogenic. As such, the epitope testing assay described herein confirms which peptides actually do bind HLA molecules. The epitope testing assay also determines how well a peptide binds and to which HLA molecule(s) the peptide binds. Determining to which HLA molecule(s) a peptide binds is important because the population is heterogeneous for HLA. Therefore, a peptide which binds many HLA is a good vaccine candidate because a vaccine incorporating that peptide will work In a broader range of the population. The peptide binding assay therefore indicates how many HLA and how well to each HLA a peptide epitope binds.

The peptide binding assay can also be coupled with direct epitope discovery methods. For example, peptide epitopes unique to infected and cancerous cells can be directly identified by producing sHLA molecules in cancerous and infected cells and then sequencing the epitopes unique to the cancerous or Infected cells. Such epitopes can then be tested for their binding to various HLA molecules to see how many HLA molecules these epitopes might bind.

In summary, a number of methods can be used to select peptides which may bind to HLA and may be immune targets. The epitope binding assay can determine whether the putative peptide target actually binds to a specific HLA molecule and how well this epitope binds the specific HLA in comparison to other known peptides that bind to the specific HLA. The epitope binding assay can also be used to screen panels of overlapping synthetic peptides to help sift through large numbers of potential vaccine/diagnostic candidates. Those peptides that bind well can then be tested for immunogenicity. Finally, the epitope binding assay can determine if a particular peptide binds multiple HLA molecules as well as the peptide's relative affinity for various HLA molecules.

**Methods of Epitope Discovery and Comparative Ligand Mapping**

As stated above, peptides utilized in the peptide binding assay can be identified by direct epitope discovery methods. For example, peptide epitopes unique to infected and cancerous cells can be directly identified by producing sHLA molecules in cancerous and infected cells and then sequencing the epitopes unique to the cancerous or infected cells. Such epitopes can then be tested for their binding to various HLA molecules to see how many HLA molecules these epitopes might bind.

Methods of epitope discovery include the following steps: (1) providing a cell line containing a construct that encodes an individual soluble class I or class II MHC molecule (wherein the cell line is capable of naturally processing self or nonself proteins Into peptide ligands capable of being loaded into the antigen binding grooves of the class I or class II MHC molecules); (2) culturing the cell line under conditions which allow for expression of the individual soluble class I or class II MHC molecule from the construct, with such conditions also allowing for the endogenous loading of a peptide ligand (from the self or non-self processed protein) into the antigen binding groove of each individual soluble class I or class II MHC molecule prior to secretion of the soluble class I or class II MHC molecules having the peptide ligands bound thereto; and (4) separating the peptide ligands from the individual soluble class I or class II MHC molecules.

Class I and class II MHC molecules are really a trimolecular complex consisting of an alpha chain, a beta chain, and the alpha/beta chain's peptide cargo (i.e. peptide ligand) which is presented on the cell surface to immune effector cells. Since it is the peptide cargo, and not the MHC alpha and beta chains, which marks a cell as infected, tumorigenic, or diseased, there is a great need to identify and characterize the peptide ligands bound by particular MHC molecules. For example, characterization of such peptide ligands greatly aids in determining how the peptides presented by a person with MHC-associated diabetes differ from the peptides presented by the MHC molecules associated with resistance to diabetes. As stated above, having a sufficient supply of an individual MHC molecule, and therefore that MHC molecule's bound peptides, provides a means for studying such diseases. Because the method of the present invention provides quantities of MHC protein previously unobtainable, unparalleled studies of MHC molecules and their important peptide cargo can now be facilitated and utilized to distinguish infected/tumor cells from uninfected/non-tumor cells by unique epitopes presented by MHC molecules in the disease or non-disease state.

The method of the present invention includes the direct comparative analysis of peptide ligands eluted from class I HLA molecules. The addition of a C-terminal epitope tag (such as a 6-HIS or FLAG tail) to transfected class I molecules has no effects on peptide binding specificity of the class I molecule and consequently has no deleterious effects on direct peptide ligand mapping and sequencing, and also does not disrupt endogenous peptide loading.

The method described further relates to a novel method for detecting those peptide epitopes which distinguish the Infected/tumor cell from the uninfected/non-tumor cell. The results obtained from the present inventive methodology cannot be predicted or ascertained indirectly; only with a direct epitope discovery method can the unique epitopes described be identified. Furthermore, only with this direct approach can it be ascertained that the source protein is degraded into potentially immunogenic peptide epitopes. Finally, this unique approach provides a glimpse of which proteins are uniquely up and down regulated in infected/tumor cells.

The utility of such HLA-presented peptide epitopes which mark the infected/tumor cell are three-fold. First, diagnostics designed to detect a disease state (i.e., infection or cancer) can use epitopes unique to infected/tumor cells to ascertain the presence/absence of a tumor/virus. Second, epitopes unique to Infected/tumor cells represent vaccine candidates. Here, we describe epitopes which arise on the surface of cells infected with HIV. Such epitopes could not be predicted without natural virus infection and direct epitope discovery. The epitopes detected are derived from proteins unique to virus infected and tumor cells. These epitopes can be used for virus/tumor vaccine development and virus/tumor diagnostics. Third, the process indicates that particular proteins unique to virus infected cells are found in compartments of the host cell they would otherwise not be found in. Thus, we identify uniquely upregulated or trafficked host proteins for drug targeting to kill infected cells.

Peptide epitopes unique to the HLA molecules of HIV infected cells can be identified by direct comparison to HLA peptide epitopes from uninfected cells by the method illustrated in the flow chart of FIG. 2. Such method has been shown to be capable of identifying: (1) HLA presented peptide epitopes, derived from intracellular host proteins, that are unique to infected cells but not found on uninfected cells, and (2) that the intracellular source-proteins of the peptides are uniquely expressed/processed in HIV infected cells such that peptide fragments of the proteins can be ' presented by HLA on infected cells but not on uninfected cells.

The method of epitope discovery and comparative ligand mapping also, therefore, describes the unique expression of proteins in infected cells or, alternatively, the unique trafficking and processing of normally expressed host proteins such that peptide fragments thereof are presented by HLA molecules on Infected cells. These HLA presented peptide fragments of intracellular proteins represent powerful alternatives for diagnosing virus infected cells and for targeting infected cells for destruction (i.e., vaccine development).

A group of the host source-proteins for HLA presented peptide epitopes unique to HIV infected cells represent source-proteins that are uniquely expressed in cancerous cells. For example, through using the methodology of the present invention a peptide fragment of reticulocalbin is uniquely found on HIV infected cells. A literature search indicates that the reticulocalbin gene is uniquely upregulated in cancer cells (breast cancer, liver cancer, colorectal cancer). Thus, the HLA presented peptide fragment of reticulocalbin which distinguishes HIV infected cells from uninfected cells can be inferred to also differentiate tumor cells from healthy non-tumor cells. Thus, HLA presented peptide fragments of host genes and gene products that distinguish the tumor cell and virus infected cell from healthy cells have been directly identified. The epitope discovery method is also capable of identifying host proteins that are uniquely expressed or uniquely processed on virus infected or tumor cells. HLA presented peptide fragments of such uniquely expressed or uniquely processed proteins can be used as vaccine epitopes and as diagnostic tools.

The methodology to target and detect virus infected cells may not be to target the virus-derived peptides. Rather, the methodology of the present invention indicates that the way to distinguish infected cells from healthy cells is through alterations in host encoded protein expression and processing. This is true for cancer as well as for virus infected cells. The methodology according to the present invention results in data which indicates without reservation that proteins/peptides distinguish virus/tumor cells from healthy cells.

In a brief example of the methodology of comparative ligand mapping utilized in the methods of the present Invention, a cell line producing Individual, soluble MHC molecules is constructed as described herein before. A portion of the transfected cell line is cocultured with a virus of interest, resulting in high-titre virus and providing infected cells. In the example provided herein the virus of interest is HIV. Alternatively, a portion of the cell line producing individual, soluble MHC molecules may be transformed to produce a tumor cell line.

The non-infected cell line and the cell line infected with HIV are both cultured in hollow-fiber bioreactors as described herein above and the soluble HLA-containing supernatant is then removed from the hollow-fiber bioreactors. The uninfected and infected harvested supernatants were then treated in an identical manner post-removal from the cell-pharm.

MHC class I-peptide complexes were affinity purified from the infected and uninfected supernatants using W6/32 antibody. Following elution, peptides were isolated from the class I molecules and separated by reverse phase HPLC fractionation. Separate but Identical (down to the same buffer preparations) peptide purifications were done for each peptide-batch from uninfected and infected cells.

Fractionated peptides were then mapped by mass spectrometry to generate fraction-based ion maps. Spectra from the same fraction in uninfected/infected cells were manually aligned and visually assessed for the presence of differences in the ions represented by the spectra. Ions corresponding to the following categories were selected for MS/MS sequencing: (1) upregulation in infected cells (at least 1.5 fold over the same ion in uninfected cells), (2) downregulation in infected cells (at least 1.5 fold over the same ion in the uninfected cells), (3) presence of the ion only in infected cells, or (4) absence of ion in infected cells that is present in uninfected cells. In addition, multiple parameters were established before peptides were assigned to one of the above categories, including checking the peptide fractions preceding and following the peptide fraction by MS/MS to ensure that the peptide of interest was not present in an earlier or later fraction as well as generation of synthetic peptides and subjection to MS/MS to check for an exact match. In addition, one early quality control step involves examining the peptide's sequence to see if it fits the "predicted motif" defined by sequences that were previously shown to be presented by the MHC molecule utilized.

After identification of the epitopes, literature searches were performed on source proteins to determine their function within the infected cell, and the source proteins were classified into groups according to functions inside the cell. Secondly, source proteins were scanned for other possible epitopes which may be bound by other MHC class I alleles. Peptide binding predictions were employed to determine if other peptides presented from the source proteins were predicted to bind, and proteasomal prediction algorithms were likewise employed to determine the likelihood of a peptide being created by the proteasome.

In accordance with the present invention, Table I lists peptide ligands that have been identified as being presented by the B*0702 class I MHC molecule in cells infected with the HIV MN-1 virus but not in uninfected cells, and also lists one peptide ligand that has been identified as not being presented by the B*0702 class I MHC molecule in cells infected with the HIV MN-1 virus that is presented in uninfected cells. As one of ordinary skill in the art can appreciate the novelty and usefulness of the present methodology in directly identifying such peptide ligands and the importance such identification has for numerous therapeutic (vaccine development, drug targeting) and diagnostic tools.

As stated above, Table I identifies the sequences of peptide ligands identified to date as being unique to HIV infected cells. Class I sHLA B*0702

# TABLE I

| ION | FRACTION | SEQUENCE | MW | OBS'D MW | SOURCE PROTEIN | START AA | ACCESSION # | CATEGORY | SEQ ID NO: |
|---|---|---|---|---|---|---|---|---|---|
| ptides Identified on Infected cells that are not present on Uninfected Cells | | | | | | | | | |
| 12.720 | 32INF | EQMFEDIISL | 1223.582 | 1223.418 | HIV MN-1, ENV | 101 | | HIV-DERIVED | 1 |
| )9.680 | 31INF | IPCLLISFL | 1017.601 | 1017.334 | CHOLINERGIC RECEPTOR, ALPHA-3 POLYPEPTIDE | 250 | | | 2 |
| i9.180 | 31INF | STTAICATGL | 936.466 | 936.360 | UBIQUITIN-SPECIFIC PROTEASE | 152 | 10720340 | | 3 |
| :0.130 | 16INF | APAQNPEL | 838.426 | 838.259 | B-ASSOCIATED TRANSCRIPT PROTEIN 3 (BAT3) | | | MHC GENE PRODUCT | 4 |
| i0.190 | 28INF | LVMAPRTVL | 998.602 | 998.396 | HLA-B HEAVY CHAIN LEADER SEQUENCE | 2 | 4566550 | MHC GENE PRODUCT | 5 |
| 9.680 | 31INF | APFI[NS]PADX | 1057.388 | | UNKNOWN, CLOSE TO SEVERAL cDNA's | | | UNKNOWN | 6 |
| 3.166 | 12INF | TPQSNRPVm | 1044.500 | 1044.333 | RNA POLYMERASE II POLYPEPTIDE A | 527 | 4505939 | RNA MACHINERY/BINDING PR | 7 |
| 4.140 | 16INF | AARPATSTL | 887.495 | 887.280 | EUK, TRANSLATION INITIATION FACTOR 4 | 1073 | Q04637 | RNA MACHINERY/BINDING PR | 8 |
| 0.650 | 16INF | MAMMAALMA | 940.413 | 939.410 | SPARC-LIKE PROTEIN | 19 | 478522 | TUMOR SUPPRESSOR GENE? | 9 |
| 490.620 | 16INF | IATVDSYVI | | 979.240 | TENASCIN-C (HEXABRACHION) | 1823 | 13639246 | TUMOR SUPPRESSOR GENE? | 10 |
| 563.640 | 16INF | SPNQARAQAAL | 1126.597 | 1126.364 | POLYPYRIMIDINE TRACT-BINDING PROTEIN 1 | 141 | 131528 | RNA MACHINERY/BINDING PR | 11 |
| | 30INF | GPRTAALGLL | 968.589 | 968.426 | RETICULOCALBIN | 4 | 4506457 | TUMOR SUPPRESSOR GENE? | 12 |
| 556.150 | 16INF | NPNQNKNVAL | 1111.586 | 1111.300 | ELAV (HuR) | 188 | 4503551 | RNA MACHINERY/BINDING PR | 13 |
| | | RPYSNVSNL | | | SBF-1 | | | | 14 |
| | | LPQANRDTL | | | MgcRacGap | | | | 15 |
| | | QPRYPVNSV | | | TCP-1 | | | | 16 |
| | | APAYSRAL | | | HSP27 | | | | 17 |
| | | APKRPPSAF | | | HMG-1 or HMG-2 | | | | 18 |
| | | AASKERSGVSL | | | histone H1 family member | | | | 19 |
| Peptides Identified on Uninfected cells that are not present on Infected cells | | | | | | | | | |
| | 16UNINF | GSHSMRY | | | MHC CLASS I HEAVY CHAIN (could derive from multiple alleles, i.e., HLA-B*0702 or HLA-G, etc.) | variable | multiple | MHC Class I Product | 20 |

was harvested for T cells infected and not infected with HIV. Peptide ligands were eluted from B*0702 and comparatively mapped on a mass spectrometer so that ions unique to infected cells were apparent. Ions unique to Infected cells (and one ligand unique to uninfected cells) were subjected to mass spectrometric fragmentation for peptide sequencing. Column 1 indicates the ion selected for sequencing, column 2 is the HPLC fraction, column 3 is the peptide sequence, column 4 is the predicted molecular weight, column 5 is the molecular weight we found, column 6 is the source protein for the epitope sequenced, column 7 is where the epitope starts in the sequence of the source protein, column 8 is the accession number, and column 9 is a descriptor which briefly indicates what is known of that epitope and/or its source protein.

The methodology used herein is to use sHLA to determine what is unique to unhealthy cells as compared to healthy cells. Using sHLA to survey the contents of a cell provides a look at what is unique to unhealthy cells in terms of proteins that are processed into peptides. The data summarized in TABLE I shows that the epitope discovery technique described herein is capable of identifying sHLA bound epitopes and their corresponding source proteins which are unique to infected/unhealthy cells.

Likewise, and as is shown in Table I, peptide ligands presented in individual class I MHC molecules in an uninfected cell that are not presented by individual class I MHC molecules in an uninfected cell can also be identified. The peptide "GSHSMRY" (SEQ ID NO:20), for example, was identified by the method of the present invention as being an individual class I MHC molecule which is presented in an uninfected cell but not In an infected cell.

The utility of this data is at least threefold. First, the data indicates what comes out of the cell with HLA. Such data can be used to target CTL to unhealthy cells. Second, antibodies can be targeted to specifically recognize HLA molecules carrying the ligand described. Third, realization of the source protein can lead to therapies and diagnostics which target the source protein. Thus, an epitope unique to unhealthy cells also indicates that the source protein is unique in the unhealthy cell.

The methods of epitope discovery and comparative ligand mapping described herein are not limited to cells infected by a microorganism such as HIV. Unhealthy cells analyzed by the epitope discovery process described herein can arise from virus infection or also cancerous transformation. In addition, the status of an unhealthy cell can also be mimicked by transfecting a particular gene known to be expressed during viral infection or tumor formation. For example, particular genes of HIV can be expressed in a cell line as described (Achour, A., et al., AIDS Res Hum Retroviruses, 1994. 10(1): p. 19-25; and Chiba, M., et al., CTL. Arch Virol 1999. 144(8): p. 1469-85.) and then the epitope discovery process performed to identify how the expression of the transferred gene modifies epitope presentation by sHLA. In a similar fashion, genes known to be upregulated during cancer (Smith, E.S., et al., Nat Med, 2001. 7(8): p. 967-72), can be transferred in cells with sHLA and epitope discovery then completed. Thus, epitope discovery with sHLA as described herein can be completed on cells infected with intact pathogens, cancerous cells or cell lines, or cells into which a particular cancer, viral, or bacterial gene has been transferred. In all these instances the sHLA described here will provide a means for detecting what changes in terms of epitope presentation and the source proteins for the epitopes.

### Example of Epitope Binding Assay Using Radiolabeled Peptide and Comparison of sHLA to Detergent Lysate-Prepared HLA

A specific assay for the A*0201T allele was used to test the feasibility of a competitive binding assay to measure the binding of defined synthetic antigenic peptides using sHLA class I molecules. A peptide derived from HBV that is known to strongly bind A*0201T was used to replace the endogenous peptide In solution. An irrelevant p53 peptide was used as a negative control in that it does not compete with the endogenous peptide for specifically binding to A*0201T.

In the reaction, different concentrations of Individual sHLA having endogenous peptide bound therein was incubated with (1) a standard concentration of HBV peptide (positive reaction), or (2) p53 peptide (negative reaction), and after incubation for 48 hours at room temperature, the sHLA complexes were immobilized on a solid support using the HLA specific antibody W6/32. The A*0201T-HBV peptide complex was detected using a highly specific antibody (mouse anti-human MHC/HBV peptide antibody 5H9/1-2H) that only recognizes this particular conformation (i.e., A*0201T with HBV peptide bound therein), and A*0201T-endogenous peptide complexes are not recognized by the antibody. To visualize the replacement event, a secondary anti-mouse antibody conjugated to HRP was used.

The assay was performed using an sHLA amount of 1.5 μg A*0201T at a final volume of 20 μl (75 ng/μl) and a final peptide amount of 2 μg at a final volume of 20 μl (100 ng/μl). After incubation, the reaction was 75x diluted, and titrating amounts of sHLA-peptide complexes were captured with the W6/32 antibody coated to an ELISA plate.

FIG. 3 illustrates that saturation of the W6/32 coated ELISA plate could be achieved using the HBV peptide, demonstrating the successful replacement of the endogenous peptide with the HBV peptide. However, no saturation could be detected using the irrelevant peptide p53, thereby supporting the specificity of HBV peptide binding to sHLA A*0201T.

In addition, the sHLA molecules used herein were compared to an HLA prepared by the prior art method of detergent lysate using the above described method. FIG. 4 is a comparison of the saturation curves of the sHLA allele A*0201T and the detergent lysate preparation of A*0201 obtained through cell extraction. This figure demonstrates that a much lower concentration of sHLA-A*0201 gives a detectable and useful signal as compared to the detergent solubilized class I A*0201. This may be because the detergent solubilized class I A*0201 is really a mixture of class I molecules, or it may be because the detergent solubilized class I does not have a full mixture of endogenous loaded peptides. The peptides In the detergent solubilized A*0201 might represent only a subset of the endogenously loaded peptides; purification of A*0201 from other class I in the lysate can bias the peptides.

However, the method of assaying epitope binding described herein above is only applicable using the A*0201T allele loaded with HBV because of the Involvement of a highly specific antibody recognizing only this specific conformation. Therefore, a more direct measurement of epitope binding to sHLA has been identified that eliminates the need to isolate and produce an antibody specific for a particular peptide bound in each class I allele. The method of Fluorescence Polarization involves labeling the peptide of interest and will be described in detail herein below.

**Epitope Binding Assay Using Fluorescence Polarization (FP)**

One of the most attractive features of binding assays is their simplicity. Developing a binding assay is to demonstrate that the labeled probe binds to the molecule of interest. The following series of criteria, however, must be met in order to validate the binding assay: (1) binding should be saturable, Indicating a finite number of binding sites; (2) the binding should have the requisite specificity, where the binding affinity, defined as the dissociation constant ($K_d$), should be consistent with values determined for physiological molecules; and (3) ligand binding should be reversible, reflecting the dynamic nature of the chemical transmission process and reaching equilibrium when the ligand association rate is equal to the dissociation rate.

However, while the execution of a routine binding assay appears trivial, the development of such an assay requires many hours of testing to establish validity. Numerous factors can influence the specificity of ligand binding. Inorganic ions can influence the attachment site of a ligand and factors such as time, temperature, pH, and peptide concentration influence the kinetic properties, and possibly the specificity, of a binding assay. Other considerations include the stability of the fluorescent dye in the incubation medium and, even more fundamentally, the biological activity of the peptide.

Fluorescein is one of the most often used molecules to produce fluorescent labels to localize antigen, receptors or other moieties with affinity for the labeled molecules, FITC (fluorescein isothiocyanate) is very fluorescent in aqueous environments and it gives high sensitivity under UV light; however, its fluorescence is pH-dependent. Its green fluorescence disappears in acid, but reappears in neutral or basic solutions. The $pK_a$ of FITC is approximately 6.5, and it gives its best fluorescent signal above pH 8. FITC has a molecular weight (MW) of 473.4; excitation and emission wavelengths at 494 nm and 520 nm, respectively; and a molar extinction coefficient of 72,000 $M^{-1}$ $cm^{-1}$ in an aqueous buffer, pH 8. Thus, FITC shifts in color from a blue-to-green fluorescent product. FITC fluorescence has a tendency to fade, but this fading may be overcome through the use of agents such as n-propyl gallate or phenylenediamine to stabilize the color. However, these reagents are toxic to living cells and cannot be used in all applications. Isothiocyanates also react with a variety of metal ions to form colored complexes. Therefore, metals such as $Fe^{3+}$, $Cu^{1+}$, and $Ag^{1+}$ should be avoided in reaction mixtures.

Fluorescence is characterized by a process of absorption of incident radiation at a wavelength, $\lambda_{abs}$, followed by the emission of radiation at another wavelength, $\lambda_{emiss}$, This behavior was first described by G. G. Stokes (1852) in the form of Stokes' Law of Fluorescence in which he stated that fluorescence (emission) always appears at a wavelength greater than the wavelength of the incident (excitation) radiation.

This behavior was successfully explained by A. Einstein (1905) using Planck's quantum hypothesis. A quantum of incident light with an energy of $E_{exdt}$ is absorbed by the fluorescent molecule raising its energy to $E_{exdt}$. This is quickly followed by a downward transition of the molecule to one of the vibration levels in the ground state, $E_{emiss}$, with the emission of a quantum of light.

The phenomenon of absorption and fluorescence can be described in terms of an energy level diagram showing the transitions between the vibration levels of the ground state ($S_0$) and the vibration levels of the first excited state ($S_1$).

In orderto describe fluorescence and fluorescence polarization an incident beam strikes a fluorescent molecule (absorption) whereupon the molecule then emits fluorescent radiation. The parallel and vertical intensities of this fluorescence are represented by $I_{II}$ and $I_1$, respectively, which serve as the components used in the experimental configuration to observe fluorescence polarization.

The primary parameter used in the field of the fluorescence polarization to describe the polarization of fluorescence is the degree of polarization, P. This parameter is a very useful way of summarizing the polarization state of fluorescence.

The equation for the degree of polarization, P is:

$$\text{Polarization} = (I_{||} - I_{\perp}) / (I_{||} + I_{\perp})$$

where $I_{\perp}$ is the intensity of the fluorescence measured in the perpendicular ($_{\perp}$) or vertical (V) direction and $I_{||}$ is the intensity of the fluorescence measured in the parallel (II) or horizontal (H) direction. These measurements are carried out using a polarizer rotated to the vertical and horizontal directions, respectively.

By using a fluorescent dye to label a small molecule, its binding to another molecule of equal or greater size can be monitored using fluorescence polarization (FP). FP operates on the principle that small molecules rotate faster than large molecules. If a molecule is labeled with a fluorophore, this rotation can be measured by exciting the fluorophore with plane polarized light and then measuring the emitted light with polarizers parallel and perpendicular to the plane of excitation to determine if it is still oriented in the same plane as it was when excited. If a fluorophore is labeled on a small molecule it will rotate in the time between excitation and emission and the light emitted will be depolarized. If the labeled molecule binds to a large molecule (effectively increasing its overall size) the molecule will not rotate in the time between excitation and emission, and the light emitted will be polarized resulting in a polarization change between the free and bound forms. For convenience, units are usually 1000 mP = P.

The maximum theoretical mP value obtainable is 500 mP. Hence any experimental value greater than this suggests an artifact within the assay. The assumed theoretical mP for fluorescein is 27. When a small free tracer is bound to a large molecule, the mP is expected to increase. A good FP assay usually has an mP change of 100 or more.

A number of factors can contribute to the lowering of the maximum obtainable polarization value. Some factors that can influence this are quenching of the fluorophore by the molecules themselves, buffer quenching, adsorption onto surfaces, rotational spin (the "propeller effect") and low affinity of interaction between the components.

Polarization methods are used to measure affinities of FITC-labeled peptide probes for purified sHLA molecules. Equilibrium results contributed to the prediction of a efficacious dose ($IC_{50}$) and clarified the strong correlation of in vitro potency to the form of the sHLA molecule used in the assay. Such results also demonstrated that equilibrium polarization measurements are feasible after optimization of assay parameters. In addition, kinetic measurements are possible with FP.

Advantages of FP measurements include: homogeneous measurements, equilibrium and kinetic binding data, plate readers available with improved sensitivity and reduced minimum volume for detection, and competitor affinity data. FP uses one label only and has a truly homogeneous format, i.e., no solid phase to prepare or suspend and no washing steps required, and rapid kinetic data can be obtained. In addition, no radioactivity is required, and the ratiometric reading resists color quenching.

Minimization of the contribution of assay components to non-specific fluorescence polarization is very important. Quality factors include purity of tracer (fluorescent peptide), purity of binder (sHLA), buffer intrinsic fluorescence and ability of buffer components such as carrier proteins to bind the tracer. Some microplate materials such as polystyrene can bind free tracer, thereby increasing the polarization.

Because of the ease with which fluorescence can be detected it is also important to know that it is the compound of interest that is being traced and not a fluorescent impurity. Thus, manufacturers provide data on the specificity and purity of their products. Tracer should be >90% labeled and >95% pure. Failure to purify free fluorophore from tracer means an increased portion of the total fluorescence will not be able to change its polarization.

The dye most commonly used for labeling peptides is fluorescein. A fluorescent labeled, substance is biologically <u>not</u> Identical to the unlabeled compound and it may not behave in a manner similar to the parent molecule.

Because large proteins, cell membranes and cellular debris scatter light, causing a net increase in total polarization, impurities should be minimized, and only highly purified binder should be used. In some cases, the impurities may be corrected in part by appropriate background subtraction, but it is preferable to minimize the contribution to signal by using purified molecules.

A mixture of protease inhibitors has to be considered if degradation occurs during incubation. A simple cocktail is often sufficient to protect the molecule, such as the use of a protease inhibitor cocktail (Gibco BRL Cat# 20012-043) dissolved in PBS pH 7.4 or a final concentration of 1 mM PMSF, 73 mM pepstatin A, and 8 mM EDTA. In the sHLA assay, highly purified sHLA molecules will be used which are also not susceptible to degradation through proteases and thus do not require the addition of protease inhibitors or calcium-chelating salts (EDTA or EGTA) to the buffer system.

In addition, buffer contribution to signal should be minimized. Increased buffer fluorescence background is due to contaminants that fluoresce at the wavelength of interest. Attention to raw materials, cleanliness of mixing and storage vessels and buffer preparation methods should reduce this to acceptable levels. High background counts due to buffer or non-fluorophore components can seriously affect the signal-to-noise ratios of an assay as well as the ultimate sensitivity of an assay.

Buffers for proteins often include carrier proteins, such as bovine albumin (BSA). Albumin may bind some fluorophores, and binding of BSA to the tracer could spuriously increase the baseline polarization, thereby reducing assay range. However, this problem can be overcome by avoiding carrier proteins or using low binding alternatives such as bovine gamma globulin (BGG).

In any case, it is useful to evaluate the contribution of buffer proteins to the net polarization of the tracer by comparing polarization in buffer with and without added protein. Alternatively, the final concentration of BSA can be reduced to minimize these effects.

Finally, likely sources of imprecision which increase the standard deviation of the assay include pipeting, instrument, buffer, tracer, and protein; each component contributes to total imprecision.

A critical feature of the recombinant sHLA molecule is the ability to load the peptide binding portion with a peptide of interest. Results obtained from acid eluted peptide pools indicate that the majority of recombinant sHLA complexes are folded around undefined "bulk peptides" derived from the cell line in which they are produced. It is necessary to replace these endogenous peptides with single, well-defined, labeled standard peptides. Endogenous sHLA molecules having endogenous peptide or no peptide bound will probably be present in a majority, but will not have any interference with the outcome of the assay. The peptide should be highly purified, as small contaminants in synthesized peptides can inhibit peptide loading.

Several peptide loading protocols have been described. A simple method involves passive loading of excess peptide in solution with sHLA. Passive loading works particularly well in the case of high-affinity peptides. For lower-affinity peptides, an increase in the molar ratio of peptide to HLA may improve loading. For each peptide, parameters such as the dose of HLA, molar ratio of peptide to HLA and peptide loading time need to be empirically determined by the investigator.

In order for the MHC class I alleles to be capable of binding peptides, recent peptide loading experiments indicated that β2-microglobulin (β-2-m) must be present. In general, MHC class I molecules are passively loaded over a several-day time course (in a range of from about 2 to about 5 days). Optimal peptide loading may vary for specific MHC class I alleles. While passively loaded complexes are generally sufficient to work with, they are not necessarily optimally loaded. Parameters and minimal requirements for peptide binding to HLA have been reported. (Khilko SN, et al. Measuring Interactions of MHC class 1 molecules using surface plasmon resonance. J Immunol Methods 183(1): 77-94 (1995); Parker KC, et at. Peptide binding to HLA-A2 and HLA-B27 isolated from Escherichia coll. Reconstitution of HLA-A2 and HLA-B27 heavy chain/beta 2-microglobulin complexes requires specific peptides. J Biol Chem. 267(8): 5451-9 (1992); Parker KC, et al. Sequence motifs important for peptide binding to the human MHC class I molecule, HLA-A2. J Immunol. 149(11):3580-7 (1992).

HLA complexes are also successfully loaded by a short alkaline stripping procedure followed by slow refolding at neutral pH. Peptide stripping can also be done in the presence of excess β-2-m under mildly acidic conditions.

There are three basic types of binding experiments: (1) saturation experiments in which a saturation curve is generated, by holding either the amount of fluorescent peptide (tracer) or sHLA (binder) constant and varying the concentration of sHLA in case of constant tracer or the labeled peptide in case of constant binder in order to determine the affinity constant ($K_d$); (2) competition experiments in which the amount of a competing, unlabeled compound for the receptor, included in the incubation, is the only variable, and the affinity (Ki) of that drug can be determined; and (3) kinetic experiments from which the forward ($k_{on}$) and reverse ($k_{off}$) rate constants of the binding process can be determined if the amount of sHLA and peptide are held constant and the time varied. The ratio of these constants provide an independent estimate of the $K_d$.

### Description of Saturation Experiments

Passive loading of excess peptide in solution with sHLA is used in the saturation assays. For each peptide, parameters such as the dose of HLA, molar ratio of peptide to HLA and peptide loading time need to be empirically determined. MHC class I molecules are passively loaded over a several-day time course (in a range of from about 2 to about 5 days). Optimal peptide loading may vary for specific MHC class I alleles.

### Titration assay to establish optimal sHLA concentration.

The assay will determine the sHLA concentration necessary to yield a sufficient peptide binding. Specific binding of various concentrations of sHLA (dose range: 0.1 nM -1000 nM) in the presence of a fixed concentration (5 nM) of fluorescent-labeled synthetic peptide should be tested. The fixed fluorescent-labeled synthetic peptide should be evaluated in preliminary experiments including biochemical considerations: the concentration of the tracer should be less than the $K_d$ and less than the concentration of available binder (sHLA) allowing peptide binding. It is recommended that the binder (sHLA) should be at a higher concentration than tracer (fluorescent-labeled synthetic peptide).

Comparison of free tracer with free fluorophore (by running free fluorescein in parallel) establishes the suitability of tracer size. If the tracer mP is significantly greater than that of the comparable fluorophore, the tracer may not be optimal for the use in FP. For adequate net polarization change, evaluation of several tracers must be conducted.

The purpose of titrating binder (SHLA) with appropriate controls is to determine the optimal concentration of binder and tracer. The acceptable range of concentrations of tracer include all concentrations giving a polarization value (in mP) near to the prescribed 27 mP. If the integration time is 100,000 microseconds, the counts per second should be at least 100,000.

Use of appropriate controls allows accurate estimation of specific polarization. The background signal is the contribution to the measurement from sources other than the fluorescent label. It is most easily seen taking a measurement on a well containing all test components except the fluorescent label. Background signals may arise from the microplate plastic, solution contaminants, leakage of light through the optical filters, or other sources generated in the instrument. If the background is highly predictable, i.e., constant from well to well, it can largely be eliminated by subtracting the signal from a control well lacking fluorescent label. Subtracting this constant signal will yield useful information from wells generating signals that are close to background levels.

Specific control groups utilized in the assay include: (1) Buffer only, (2) Tracer only, (3) Protein only and (4) Protein + Tracer. The purpose of each of the specific control groups is discussed in detail herein below.

The "Buffer only" control indicates the contribution of buffer alone to the S and P signals, especially when interfering molecules are present in the buffer. Since the binder may contribute to net signal, binder without tracer serves as a proper control and is used as background subtraction for "Tracer only". For multiple concentrations of binder, each should have the "Buffer only" control.

In the "Tracer only" control, S and P values for free tracer are background-subtracted with "Buffer only" controls and used for G factor calculation. G factors are calculated using the assumed theoretical mP (27 for fluorescein). S (parallel) and P (perpendicular) are the background subtracted intensity measurements when the polarizers are in parallel or perpendicular direction.

$$\text{G factor} = \frac{S}{P} * \frac{\left(1 - \frac{27}{1000}\right)}{\left(1 + \frac{27}{1000}\right)}$$

G should be a very stable value which corrects for the contribution of the measurement pathway to the observed total polarization. Optical pathways, particularly those with reflective components, pass light of different polarization with varying efficiencies. Instrumental elements that impact this correction factor include the dichroic mirror, excitation and emission filters, polarizing filters and attenuators. Other elements that influence the G factor include the assay plate and buffer/assay components.

Essentially the G factor functions as a scaling (correction) factor, taking relative polarization measurements and making them appear absolute (relative to a known standard). The G factor is typically a value ranging from 0.8 to 1.2. Obtaining a G factor very different from 1 suggests that the filters and dichroic mirror may not be optimized for fluorescence polarization, although meaningful results may be obtained.

Once a G factor has been determined, it can be entered into the associated fluorescence polarization method. The calculated mP from the Criterion Host software will now report "corrected" mP values. Using the established G factor, calculate the mP value for the free tracer. The mP values are generally obtained by subtracting the mean S and P background values from the individual S and P values of the free tracer. As a control, the free fluorophore should have an mP value close to the theoretical value and serves as the minimal polarization value ($mP_{min}$).

$$mP = \frac{S - (P * G)}{S + (P * G)} * 1000$$

The "Protein only" control indicates contribution of light scattering by the specific protein binder. This is used as background subtraction for "Protein + Tracer". Since several concentrations of protein will be used, each should be tested in the absence of tracer.

For "Protein + Tracer", the S and P values are background subtracted with "Protein only" controls to determine the maximal mP for a given concentration. For background subtraction, the mean S and P "Protein only" values are calculated, and the appropriate mean from individual S and P values of wells containing "Protein + Tracer" are subtracted. The calculated G factor is used.

$$mP = \frac{S - (P * G)}{S + (P * G)} * 1000$$

As an additional check on the system, it is advisable to re-read the plate in the Fluorescence Intensity mode. If the same amount of tracer is present in each well, then there should be equal intensity values across the plate in the Fluorescence Intensity mode. Re-reading the plate in intensity mode allows evaluation of the extent of quenching. Quenching effects can affect the ultimate sensitivity of a fluorescence-based assay. The comparison of the molar fluorescence intensity of the fluorophore-labeled peptide and the fluorophore itself in free solution can be used to determine the degree of quenching caused by the chemical coupling process itself. If there is no quenching, the signal for 1 nM fluoresceinated peptide should be the same as that of the free fluorescein. It is not expected that fluorescein coupled to another molecule would be more fluorescent than free fluorescein, so this could indicate that the tracer may have an incorrect concentration assigned. Fluorescence polarization often results in the loss of about 10-90% of fluorescence intensity. This in itself may reduce the sensitivity of fluorescence polarization as opposed to direct intensity measurements.

**Method of Epitope Binding Assay Using Flouorescence Polarization**

The FITC-labeled peptides are prepared by dissolving the lyophilized powder in 90% Acetonitrile/10% water to a final concentration of 0.25 mM (this measurement should be as precise as possible). It is important to make sure everything is gone into solution. If there is still precipitate present, Incubate overnight at room temperature with shaking. Seal the tube with parafilm and protect from light. (Some peptides will dissolve only by adding $NH_4HCO_3$ at a final concentration of 25 mM to convert the acidic environment Into a more basic state).

The FITC labeled peptide solution is divided into 800 μl aliquots and added to sealable screw cap tubes. The aliquots are then lyophilized overnight at room temperature. Each tube should finally contain 0.2 μmol of peptide. Each tube is closed tightly, and parafilm is used to wrap the top. The tube is then labeled appropriately and stored at -80°C until use.

For an adequate net polarization change, several FITC-labeled peptide tracers are evaluated. For optimal evaluation of FP data, 5 independent reaction types need to be mixed, as described in Table II.

**TABLE II**

| | | | |
|---|---|---|---|
| A | Reaction. Mix | "Protein + peptide Tracer" | sHLA; β-2-m; pFITC; PBS; (BGG) |
| B | Protein only control | "Protein only" | sHLA; β-2-m; PBS; (BGG) |
| C | Labeled peptide only control | "Free Peptide Tracer only" | pFITC; β-2-m; PBS; (BGG) |
| D | Free FITC tracer only control | "Free FITC Tracer only" | FITC; β-2-m; PBS; (BGG) |
| E | Buffer only control | "Buffer only" | β-2-m; PBS; (BGG) |

Multiple concentrations of sHLA are tested for specific binding in a range of 0.1-1000 nM. A first study may include rather broad concentration ranges for both tracer and binder whereas a follow-up test may use only one concentration of tracer and a tightly spaced limited dilution series of the binder.

| | | |
|---|---|---|
| HC (A*0201T) | 273 aa | (31,495) |
| N-glycosylation | - | (3,000) |
| β-2-m | | 99 aa<br>(11,731) |
| Peptide (9mer) | 9 aa | (1,000) |
| **Complex** | **381 aa** | **(47,226)** |

(continued)

| | |
|---|---|
| 47.2 μg/ml | 1 μM |
| 47.2 ng/ml | 1 nM |

In order for the MHC class I alleles to be capable of binding peptides, recent peptide loading experiments indicated that additional β2-microglobulin must be present. The addition of an extra amount of β-2-m in a ratio of β-2-m:sHLA between about 0.5 to about 2.0 are reasonable. Desirably, a ratio of β-2-m:sHLA of about 1.0 is utilized in the beginning and then adjusted if required.

| **β-2-m** | **99 aa** | **(11,731)** |
|---|---|---|
| | 11.7 μg/ml | 1 μM |
| | 11.7 ng/ml | 1 nM |

Suggested dilutions of sHLA and β-2-m are as shown in Table III:

**TABLE III**

| | sHLA dilutions | | β-2-m dilutions | |
|---|---|---|---|---|
| 1 | 50,000 ng/ml | 1060 nM | 12,402 ng/ml | 1060 nM |
| 2 | 10,000 ng/ml | 212 nM | 2,480 ng/ml | 212 nM |
| 3 | 5,000 ng/ml | 106 nM | 1,240 ng/ml | 106 nM |
| 4 | 1,000 ng/ml | 21.2 nM | 248 ng/ml | 21.2 nM |
| 5 | 500 ng/ml | 10.6 nM | 124 ng/ml | 10.6 nM |
| 6 | 250 ng/ml | 5.3 nM | 62 ng/ml | 5.3 nM |
| 7 | 125 ng/ml | 2.65 nM | 31 ng/ml | 2.65 nM |
| 8 | 62.5 ng/ml | 1.32 nM | 15.5 ng/ml | 1.32 nM |
| 9 | 31.25 ng/ml | 0.66 nM | 7.75 ng/ml | 0.66 nM |
| 10 | 15.6 ng/ml | 0.33 nM | 3.88 ng/ml | 0.33 nM |
| 11 | 7.8 ng/ml | 0.16 nM | 1.94 ng/ml | 0.16 nM |
| 12 | 3.9 ng/ml | 0.08 nM | 0.97 ng/ml | 0.08 nM |

To prepare 2x dilutions of sHLA + excess β-2-m, a total volume of 550 μl is sufficient to perform 5 independent measurements for A ("Protein + Peptide tracer") and B ("Protein only") with a backup volume of 50 μl. The used dilution scheme will use up 81.4 μg of sHLA and 20.2 μg β-2-m for the (A) & (B) reactions.

PBS pH 7.4 is used as the buffer. Optionally, 0.05% (0.5 mg/ml) bovine gamma globulin (BGG) may be used as supplement to prevent non-specific binding of protein on tube walls or as carrier protein. When using BGG, prepare a volume of 33 ml of freshly mixed PBS/0.05% BGG (16.5mg BGG/33 ml PBS).

To prepare the 2x dilutions, twelve "non-stick" 1.5 ml tubes are labeled with the numbers 1-12. Table IV below describes the dilutions. Each tube is mixed thoroughly after adding sHLA/β-2-m. Careful pipetting is required.

The first tube (tube #1) should originally contain 814 μl total volume with a sHLA concentration of 100,000 ng/ml and a β-2-m concentration of 24,804 ng/ml. Therefore, the amounts of sHLA and β-2-m to be added to tube #1 are calculated, and then the tube is filled up to 814 μl.

$$\mu l\ \text{sHLA to add} = \frac{100\ \mu g/ml\ \text{final conc.} * 814\ \mu l\ \text{total volume}}{[\mu g/ml\ \text{stock sHLA}]}$$

$$\mu l\ b2m\ to\ add = \frac{24.8\ \mu g/ml\ final\ conc. * 814\ \mu l\ total\ volume}{[\mu g/ml\ stock\ b2m]}$$

| TABLE IV | | | | |
|---|---|---|---|---|
| Tube # | Volume of PBS/0.05% BGG to add | Tube # or stock to use | Volume of sHLA/β-2-m sample to add | 2x dilutions (ng/ml) |
| 1 | **see above** | **see above** | **see above** | 100,000 / 24,804 |
| 2 | 1056 μl | Tube 1 | 264 μl | 20,000 / 4,961 |
| 3 | 770 μl | Tube 2 | 770 μl | 10,000 / 2,480 |
| 4 | 880 μl | Tube 3 | 220 μl | 2,000/496 |
| 5 | 550 μl | Tube 4 | 550 μl | 1,000 / 248 |
| 6 | 550 μl | Tube 5 | 550 μl | 500 / 124 |
| 7 | 550 μl | Tube 6 | 550 μl | 250/62 |
| 8 | 550 μl | Tube 7 | 550 μl | 125/31 |
| 9 | 550 μl | Tube 8 | 550 μl | 62.5/15.5 |
| 10 | 550 μl | Tube 9 | 500 μl | 31.25/7.75 |
| 11 | 550 μl | Tube 10 | 550 μl | 15.6/3.88 |
| 12 | 550 μl | Tube 11 | 550 μl | 7.8/1.94 |

To prepare 2x control dilutions of excess β-2-m only, a total volume of 800 μl is sufficient to make up the rest of the controls necessary for C ("Free peptide tracer only"), D ("Free FITC tracer only") and E ("Buffer only") with a backup volume of 50 μl. The used dilution scheme will use about 29.4 μg β-2-m for the (C), (D) & (E) reaction.

To prepare the 2x dilutions, twelve "non-stick" 1.5 ml tubes are labeled with the numbers 1-12. Table V describes the dilutions. Each tube is mixed thoroughly after adding sHLA/β-2-m, Careful pipetting is required.

The first tube (tube #1) should originally contain 1184 μl total volume with a β-2-m concentration of 24,804 ng/ml. The amount of β-2-m to be added to tube#1 is calculated, and then the tube is filled to 1184 μl.

$$\mu l\ b2m\ to\ add = \frac{24.8\ \mu g/ml\ final\ conc. * 1184\ \mu l\ total\ volume}{[\mu g/ml\ stock\ b2m]}$$

| TABLE V | | | | |
|---|---|---|---|---|
| Tube # | Volume of PBS/0.05% BGG to add | Tube # or stock to use | Volume of b-2-m sample to add | 2x dilutions (ng/ml) |
| **1** | **see above** | **see above** | **see above** | 24,804 |
| 2 | 1536 μl | Tube 1 | 384 μl | 4,961 |
| 3 | 1120 μl | Tube 2 | 1120 μl | 2,480 |
| 4 | 1280 μl | Tube 3 | 3,20 μl | 496 |
| 5 | 800 μl | Tube 4 | 800 μl | 248 |
| 6 | 800 μl | Tube 5 | 800 μl | 124 |

(continued)

| TABLE V | | | | |
|---|---|---|---|---|
| Tube # | Volume of PBS/0.05% BGG to add | Tube # or stock to use | Volume of b-2-m sample to add | 2x dilutions (ng/ml) |
| 7 | 800 μl | Tube 6 | 800 μl | 62 |
| 8 | 800 μl | Tube 7 | 800 μl | 31 |
| 9 | 800 μl | Tube 8 | 800 μl | 15.5 |
| 10 | 800 μl | Tube 9 | 800 μl | 7.75 |
| 11 | 800 μl | Tube 10 | 800 μl | 3.88 |
| 12 | 800 μl | Tube 11 | 800 μl | 1.94 |

Finally, a stock solution of FITC-labeled peptide tracer (pFITC) is prepared using a pre-measured tube (0.2 μmol) and stored at -80°C. 20 μl of DMSO is added to 0.2 μmol of FITC-labeled peptide powder pre-measured in a microtube to receive a stock concentration of 10 mM and pipetted up and down until the tracer is completely dissolved. Optionally, 20 μl of DMF may be used for peptides containing methionine (M) which are known to oxidize more likely in DMSO than DMF. To protect from moisture, the FITC-labeled peptide and DMSO are allowed to equilibrate to room temperature before opening.

To obtain a 100 μM working dilution, 980 μl of PBS is added to the original DMSO stock, and then the working concentration is further diluted to a 1 μM solution (10 μl working dilution to 990 μl PBS). The 100 μM working dilution is aliquoted and stored at -25°C.

Acceptable concentrations of FITC-labeled peptides in FP experiments are in a 0.75 to 50 nM range. A mP range of free FTTC tracer is expected to be between 30-50 mP. Intensities for parallel and perpendicular fluorescence for the concentration range of 0.75 to 50 nM FITC is 0.3 to 30 $10^6$ cps. Unpolarized fluorescence intensity will be 10× over the polarized intensities. An optimal starting concentration of FITC-labeled peptide would be 5 nM. Consider running more than one concentration of tracer initially.

To prepare 20 ml of 5 nM pFITC used in (A) ("Protein + Peptide tracer") and (C) ("Free peptide tracer only"), 100 μl of the 1 μM pFITC is added to 19.9 ml buffer. Free peptide tracer is compared with free fluorophore by running free FITC in parallel to establish the suitability of the tracer and as a control if the correct concentration was assigned to the peptide tracer. If there is no quenching the signal for the selected fluoresceinated peptide concentration should be the same as that of the FITC control.

A stock solution of FITC is prepared using pre-measured FITC (Fluorescein Isothiocyanate) Microtubes (Pierce # 51004; 6 × 1 mg). (1 mg/ml is 2.112 mM). FITC has a molecular weight (MW) of 473.4. 211.2 μl of DMSO is added to 1 mg of FITC powder pre-measured in a microtube to receive a stock concentration of 10 mM. The FITC labeling reagent is reconstituted by puncturing the foil and adding 211.2 μl of DMSO and pipetting up and down until the FITC is completely dissolved. To protect from moisture, the FITC and DMSO are allowed to equilibrate to room temperature before opening.

To obtain a 100 μM working dilution, 980 μl of PBS is added to the original DMSO stock. The working concentration is further diluted to a 1 μM solution (10 μl working dilution to 990 μl PBS). The 100 μM working dilution is aliquoted and stored at -25°C.

To prepare 10 ml of 5 nM pFITC used in (D) ("Free FITC tracer only"), 50 μl of the 1 μM pFITC is added to 9.95 ml buffer.

To start the peptide exchange reaction, mix 50 μl of each solution prepared above according to the scheme shown in FIG. 5. 50 μl buffer (PBS/(BGG) is added in row 1-12 of B & E. 50 μl of FITC solution is added in row 1-12 of D, and 50 μl of pFITC is added in row 1-12 of A & C. The serial dilutions of the β-2-m series are added only to C, D & E starting with 12 and ending with 1. Finally, the serial dilutions of the sHLA/β-2-m series are added to A & B, also starting with 12 and ending with 1. The dilutions are then kept at 4°C in the dark.

The meniscus are checked to make sure they are uniform and are evened out by gentle tapping if necessary. Air bubbles, which may be present in some wells, are removed.

The usual incubation time for a peptide exchange reaction is about 48 hours. However, to determine optimal binding times, the course of binding should be observed, by reading the plate several times during incubation. Start the first reading at t=0 hours.

A fluorescence polarization detection method is prepared with the following parameters:

**Lamp:** continuous lamp
**Plate format:** as appropriate for the plate to be evaluated
**Switch polarization** after each well
**Select wells:** specify all the wells to be read
**G factor:** 1
**Z Height:** 1 mm
**Filters:** fluorescein excitation and emission
(also use the fluorescein dichroic in the top optics head)
**Timing, Continuous Lamp:**

Readings per Well: 1
Integration Time:100,000 μsec

**Raw Data Units:** counts/sec
**Attenuator mode:** out
**Polarizers:** excitation polarizer in the S position emission polarizer as dynamic
**PMT setup:** SmartRead, sensitivity 2
**Plate agitation:** none
**Kinetic timing:**

Delay Before First Read: 0
Delay Between Reads: 0
Number of Reads: 1.

**Plate Settling Time:** 25 ms

The plate is re-read in **Fluorescence Intensity mode** to determine the degree of quenching caused by the chemical coupling process of sHLA and peptide. Without quenching and equal amount of tracer present in each well, the intensity values across the plate should be the same. Note that fluorescence intensity reaches a 10-90% higher signal than fluorescence polarization.

A fluorescence intensity detection method is then prepared with the following parameters:

**Optics:** top, continuous lamp
**Plate format:** as appropriate for the plate to be evaluated
**Select wells:** specify all the wells to be read
**Z Height:** 1mm
Filters: fluorescein excitation and emission
(also use the fluorescein dichroic in the top optics head)
**Timing, Continuous Lamp:**

Readings per Well: 1
Integration Time:100,000 μsec

**Raw Data Units:** counts/sec
**Attenuator mode:** out
**Polarizers:** none
**PMT setup:** SmartRead, sensitivity 2
**Plate agitation**: none
**Kinetic timing:**

Delay Before First Read: 0
Delay Between Reads: 0
Number of Reads: 1.

**Plate Settling Time:** 25 ms

After reading the plate, the plate is covered with a lid and sealed with parafilm. To protect from light, the plate is additionally covered with aluminum foil. The plate is then incubated at assigned temperature until next reading. Each reading is recorded as follows:

| Date: | Time: | No. | Incubation period | Current incubation time |
|---|---|---|---|---|
| | | $t_1$ | 0 hours | 0 hours |
| | | $t_2$ | hours | hours |
| | | $t_3$ | hours | hours |
| | | $t_4$ | hours | hours |
| | | $t_5$ | hours | hours |
| | | $t_6$ | hours | hours |
| | | $t_7$ | hours | hours |
| | | $t_8$ | hours | hours |
| | | $t_9$ | hours | hours |
| | | $t_{10}$ | hours | hours |

The data is evaluated using a spread-sheet program, such as Microsoft's EXCEL™. The net increase in polarization is determined upon addition of sHLA to the peptide.
The "Free peptide tracer only" values provide the basis to calculate the G factor. "Tracer only" gives the lowest attainable mP (minimum tracer binding) not using a G factor to correct values to a theoretically assumed value. The "Buffer only" control provides a background control, indicating the contribution of buffer only to the S and P signals, especially when interfering molecules are present in the buffer. The background signal is the contribution to the measurement from sources other than the fluorescent label. It is most easily seen taking a measurement on a well containing all test components except the fluorescent label. Background signals may arise from the microplate plastic, solution contaminants, leakage of light through the optical filters, or other sources generated in the instrument. S and P values for "Free peptide tracer only" and "Free FITC tracer only" are background-subtracted with "Buffer only" controls before calculating mP and G factor.

If there are several concentrations of (unlabeled) components present (i.e., multiple β-2-m concentrations), there should be a background well for each concentration. Experiments with homogenous buffer systems are background corrected by subtracting the mean values.

G factors are calculated using the assumed theoretical mP (27 for fluorescein). S (parallel) and P (perpendicular) are the background subtracted intensity measurements when the polarizers are in parallel or perpendicular direction.

$$\text{G factor} = \frac{S}{P} \cdot \frac{\left(1 - \frac{27}{1000}\right)}{\left(1 + \frac{27}{1000}\right)}$$

Using the calculated G factor will adjust all results to the assumed theoretical mP of 27 as the lowest attainable mP value (minimum tracer binding). The signal to background ratios are determined to assure the quality of the G factor.
"Free peptide tracer only" / "Buffer only"
and
"Free FITC tracer only" / "Buffer only"
A representative value for signal/background can be calculated from the Parallel (S) value of signal "Free peptide tracer only" to background "Buffer only" values. Ideally, signal to background values of at least 10-fold should be targeted. Errors become usually large at low concentration as background noise dominates the intensities.

Noise relates to the uncertainty in measurements and usually determines the ultimate sensitivity of an instrument. To determine signal to noise ratios, the mP values are divided by the standard deviation where the mP value corresponds to signal and the imprecision (standard deviation) corresponds to the "noise". Ideally, signal to noise values of at least three times the background standard deviation should be targeted.

As additional check on the system, it is advisable to re-read the plate in the Fluorescence Intensity mode. Re-reading the plate in intensity mode allows evaluation of the extent of quenching. Quenching effects can affect the ultimate sensitivity of a fluorescence-based assay.

The comparison of the molar fluorescence intensity of the fluorophore-labeled peptide and the fluorophore itself in free solution can be used to determine the degree of quenching caused by the chemical coupling process itself. Compare "Free peptide tracer only" fluorescence intensities with "Free FITC tracer only" fluorescence intensities re-read in fluorescence intensity mode. If there is no quenching, the signal for 1 nM fluoresceinated peptide should be the same as that of the free fluorescein. It is not expected that fluorescein coupled to another molecule to be more fluorescent than free fluorescein, so this could indicate that the tracer may have an incorrect concentration assigned. In addition, the obtained fluorescence intensity results are confirmed by G factor comparison between "Free peptide tracer only" and "Free FITC tracer only".

The S and P "Protein + Peptide tracer" values determine the highest attainable mP (maximum tracer binding) for a chosen concentration. Before calculating mP, the background is subtracted with "Protein only" controls, and the calculated G factor is used.

The "Protein only" control indicates the contribution of light scattering by the specific protein binder. Since several concentrations of protein will be used, each should be tested in the absence of tracer.

$$mP = \frac{S - (P * G)}{S + (P * G)} * 1000$$

A dose-response curve will be obtained by plotting bound fluorescence values (mP) against total concentration of the sHLA molecule on a log-log plot. There should be a plateau effect, with supraoptimal concentrations of binder yielding no further increase in mP. Data points are fitted to the dose-response model using the dose-response equation. Reproducibility can be shown by averaging the value from 3 independent measurements.

No binding should be detected in the case of using an irrelevant fluorescent-labeled MHC molecule. No binding should be detected in the case of using an irrelevant fluorescent- labeled peptide.

Imprecision is the standard deviation of the mean of each group of mP values. This should generally be less than 10 mP. The signal to background ratios is determined to assure the quality of the results.

"Protein + Peptide tracer" / "Protein only"

A representative value for signal/background can be calculated from the Parallel (S) value of signal ("Protein + Peptide tracer") to background ("Protein only") values. Ideally, signal to background values of at least 10-fold should be targeted.

The assay range as the change in polarization is calculated by subtracting the mean mP of ("Free peptide tracer only") from the mean mP of the ("Protein + Peptide tracer"). Ideally, the net change in polarization should be greater than 70 mP. Maximum measurable binding is determined to be the ("Protein + Peptide tracer") values background subtracted with ("Protein only") controls (maximum sHLA and tracer binding allowed, which gives a high mP) minus the "Free peptide tracer only" (minimum tracer binding, which gives a very low mP). The difference between these two conditions is the maximum delta mP.

Each background-corrected mP value obtained is normalized at a specific sHLA concentration by dividing by the maximum delta mP. To calculate % of maximum binding, multiply by 100%. Optimal sHLA concentration is chosen by comparing the results according to their best signal-to-background ratio and highest polarization value.

Given milligram to gram quantities of sHLA for the many HLA alleles in the population, it is possible to automate fluorescent epitope binding assays with sHLA and thousands to millions of various peptides. Robotics or other high throughput methods introduce various peptides to the various SHLA molecules, incubate at the time and temperatures described herein, and determine those peptides that bind to a given sHLA molecule and the relative affinity of those peptides for a sHLA molecule. For example, all the possible overlapping peptides in 50 different viral pathogens could be synthesized and tested for their binding to 50 different sHLA molecules. The resulting data would provide a database of those viral peptides that bind to HLA molecules for possible presentation to the immune response. Such data would be useful for viral vaccine discovery. In a similar test, all peptides encoded by genes known to be upregulated in tumor cells are tested for their binding to sHLA molecules. The resulting data would indicate peptides for potential use in tumor vaccines. Finally, all human proteins could have overlapping peptides synthesized and tested for binding to sHLA molecules. The resulting data, combined with gene expression studies, is useful in determining those epitopes which are involved in autoimmune disorders. The resulting data could then be used to design therapies and diagnostics.

**Results from Epitope Binding Assay using Flouorescence Polarization**

Methods for published peptide binding assays with detergent lysate HLA or with bacterial produced HLA typically incubate peptides and HLA at room temperature. FIG. 6 illustrates that incubating various concentrations of sHLA with a fixed concentration of peptide at room temperature requires 10,000 to 100,000 picomoles to obtain significant binding of fluorescent peptide as detected by fluorescent polarization. Baseline levels of FP are approximately 20mp while maximum FP at room temperature incubation is approximately 70; a difference of 50 is the minimum required to differentiate between a postive and negative binding peptide.

By comparison to the incubation of peptide and sHLA at room temperature, as shown in FIG. 7, it can be seen that heating the sHLA to 54° Celsius for 45 minutes followed by incubation of the sHLA with the same peptide at 4° Celsius produces a much stronger FP signal at the same concentrations of sHLA. The difference between background and the strongest signal was approximately 130 mp. A greater difference between the baseline and the positive allows us to use less sHLA to get a signal difference of 50 mp and also allows the assay to provide greater differentiation among the various binding affinities of different peptides.

FIG. 8 demonstrates that sHLA molecules bind fluorescent peptides in a manner specific to the sHLA being tested. In this Figure, the sHLA molecule A*0201 was tested for its ability to bind 5 peptides specific for A*0201 (+P1 to +P5) and five peptides specific for the HLA molecule B*2705. The sHLA A*0201 was heated to 54°C for 45 minutes and then added to a reaction mixture at 4°C that contained the various peptides. The data shows that the sHLA-A*0201 specifically binds A*0201 peptides as detected by FP.

Fig. 9 demonstrates that sHLA A*0201 at a concentration of 50 micrograms per milliliter bind peptides specific for A*0201. Furthermore, this data demonstrates that most peptides specific for A*0201 bind to their maximum extent within 24 hours. This assay shows that peptides bind to the sHLA molecules in a specific way with an FP difference of up to 200 mp. We have therefore identified reaction conditions that quickly detect a peptide's ability to bind sHLA molecules. The strong FP difference allows us to compare peptide binding affinities of various peptides for the sHLA molecules.

In the previous figures of this application, we demonstrate that A*0201 specific peptide ligands bind to sHLA A*0201. In FIG. 10, we add a peptide that is also specific for A*0201 (A2 peptide (KLGEFYNQMM) (SEQ ID N0:21)) This "cold" competitor peptide is mixed with the "hot" fluorescent peptide (peptide P5, having the sequence ALMDKVLK (FITC)V (SEQ ID NO:22)) at various concentrations of the cold peptide. The sHLA which has been heated to 54 degrees Celsius for 45 minutes is added to the mixture of peptides which is at 4 degrees Celsius. The mixture is then incubated at 4 degrees. In FIG. 10 it can be seen that increasing concentrations of the cold peptide prevent the hot peptide from binding to sHLA A*0201. The less the hot peptide binds, the less FP is obtained. Thus, we demonstrate that peptides specific for sHLA A*0201 can compete with labeled peptides specific for A*0201. In this way we can determine the comparative affinity of various peptides for A*0201: the lower the concentration needed to compete the hot peptide means that the cold competitor has a higher affinity.

Thus, in accordance with the present invention, there has been provided a method for assaying epitope binding to HLA that includes methodology for producing and manipulating Class I and Class II MHC molecules from gDNA as well as methodology for directing discovering epitopes unique to infected or tumor cells that fully satisfies the objectives and advantages set forth herein above.

## Claims

1. A method of assaying a peptide for binding to an individual soluble MHC class I molecule, the method comprising the steps of:

    providing a peptide of interest;
    providing a pool of individual soluble MHC class I trimolecular complexes, each trimolecular complex comprising a recombinant, soluble MHC class I allele, beta-2-microglobulin, and endogenously loaded peptide, by:

        obtaining genomic DNA or cDNA encoding at least one MHC class I molecule;
        identifying an MHC class I allele in the genomic DNA or cDNA;
        PCR amplifying the MHC class I allele in a locus specific manner to produce a PCR product that encodes a truncated, soluble form of the individual MHC class I allele;
        cloning the PCR product into an expression vector, thereby forming a construct that encodes the individual soluble MHC class I allele;
        transfecting a mammalian cell line to provide a mammalian cell line expressing a construct that encodes a recombinant individual soluble MHC class I allele, wherein the mammalian cell line is able to naturally process proteins into peptide ligands for loading into antigen binding grooves of MHC class I molecules;
        culturing the mammalian cell line under conditions which allow for expression of the recombinant individual MHC class I allele from the construct, such conditions also allowing for endogenous loading of a peptide ligand into the antigen binding groove of each individual soluble MHC class I allele in the presence of beta-2-microglobulin to form the individual soluble MHC class I trimolecular complexes prior to secretion of the individual soluble MHC class I trimolecular complexes from the cell; and
        isolating the pool of individual soluble MHC class I trimolecular complexes, wherein such isolation step does not result in selective purification of particular MHC class I trimolecular complexes having certain peptides loaded therein such that the pool of individual soluble MHC class I trimolecular complexes is not biased in the peptides loaded therein;

mixing the peptide of interest with the individual soluble class I trimolecular complexes; and
identifying at least one individual soluble MHC class I trimolecular complex wherein the peptide of interest has displaced an endogenously loaded peptide and competitively bound to the soluble MHC class I allele and beta-2-microglobulin to form the trimolecular complex.

**2.** The method of claim 1 further comprising the step of treating the pool of individual soluble MHC class I trimolecular complexes under conditions that cause the endogenous peptides to be released prior to mixing the peptide of interest with the pool of individual soluble MHC class I trimolecular complexes.

**3.** The method of claim 2 wherein the step of treating the pool of individual soluble MHC class I trimolecular complexes involves heating the pool of individual soluble MHC class I trimolecular complexes to cause the the endogenous peptides to be released.

**4.** The method of claim 1 wherein, in the steps of providing the peptide of interest and identifying at least one individual soluble MHC class I trimolecular complex wherein the peptide of interest has displaced an endogenously loaded peptide, the peptide of interest is labeled to allow identification of the at least one individual soluble MHC class I trimolecular complex from unbound peptide of interest.

**5.** The method of claim 4 wherein the peptide of interest is labeled with a radiolabel or a fluorescent label.

**6.** The method of claim 5 wherein, in the step of identifying at least one individual soluble MHC class I trimolecular complex wherein the peptide of interest has displaced an endogenously loaded peptide, the peptide of interest is labeled with a fluorescent label, and the at least one individual soluble MHC class I trimolecular complex is identified by fluorescence polarization.

**7.** The method of claim 1 wherein the construct further encodes a tag which is attached to the individual soluble MHC class I allele and aids in isolating the pool of individual soluble MHC class I trimolecular-complexes.

**8.** The method of claim 7 wherein the tag is selected from the group consisting of a HIS tail and a FLAG tail.

**9.** The method of claim 1 wherein, in the step of providing a peptide of interest, the peptide of interest is identified by a method for identifying at least one endogenously loaded peptide ligand that distinguishes an infected cell from an uninfected cell, the method comprising the steps of:

providing an uninfected cell line containing a construct that encodes an individual soluble MHC class I allele, the uninfected cell line being able to naturally process proteins into peptide ligands capable of being loaded into antigen binding grooves of MHC class I molecules;
infecting a portion of the uninfected cell line with at least one of a microorganism, a gene from a microorganism or a tumor gene, thereby providing an infected cell line;
culturing the uninfected cell line and the infected cell line under conditions which allow for expression of the recombinant individual soluble MHC class I allele from the construct, such conditions also allowing for endogenous loading of a peptide ligand in the antigen binding groove of each recombinant individual soluble MHC class I allele in the presence of beta-2-microglobulin to form individual soluble MHC class I trimolecular complexes prior to secretion of the individual soluble MHC class I trimolecular complexes from the cell;
isolating the secreted individual soluble MHC class I trimolecular complexes from the uninfected cell line and the infected cell line;
separating the endogenously loaded peptide ligands from the individual soluble MHC class I trimolecular complexes from the uninfected cell line and separating the endogenously loaded peptide ligands from the individual soluble MHC. class I trimolecular complexes from the infected cell line;
isolating the endogenously loaded peptide ligands from the uninfected cell line and the endogenously loaded peptide ligands from the infected cell line;
comparing the endogenously loaded peptide ligands isolated from the infected cell line to the endogenously loaded peptide ligands isolated from the uninfected cell line; and
identifying at least one endogenously loaded peptide ligand presented by the individual soluble MHC class I allele on the infected cell line that is not presented by the individual soluble MHC class I allele on the uninfected cell line.

**10.** The method of claim 9 further comprising the step of identifying a source protein from which the at least one endogenously loaded peptide ligand presented by the individual soluble MHC class I trimolecular complex on the infected cell line and not presented by the individual soluble MHC class I trimolecular complex on the uninfected cell line is obtained.

**11.** The method of claim 9 wherein, in the step of identifying at least one endogenously loaded peptide ligand presented by the individual soluble MHC class I trimolecular complex on the infected cell line but not on the uninfected cell line, the at least one endogenously loaded peptide ligand is obtained from a protein encoded by at least one of the microorganism, the gene from a microorganism or the tumor gene with which the cell line was infected to form the infected cell line.

**12.** The method of claim 9 wherein, in the step of identifying at least one endogenously loaded peptide ligand presented by the individual soluble MHC class I trimolecular complex on the infected cell line but not on the uninfected cell line, the at least one endogenously loaded peptide ligand is obtained from a protein encoded by the uninfected cell line.

**13.** The method of claim 12, wherein the protein encoded by the uninfected cell line from which the at least one endogenously loaded peptide ligand is obtained has increased expression in a tumor cell line.

**14.** The method of claim 9 wherein, in the step of infecting a portion of the uninfected cell line, the portion of the uninfected cell line is infected with HIV.

**15.** The method of claim 1 wherein, in the step of providing a peptide of interest, the peptide of interest is identified by a method for identifying at least one endogenously loaded peptide ligand that distinguishes an infected cell from an uninfected cell, the method comprising the steps of:

providing an uninfected cell line containing a construct that encodes an individual soluble MHC class I allele, the uninfected cell line being able to naturally process proteins into peptide ligands capable of being loaded into antigen binding grooves of MHC class I molecules;
infecting a portion of the uninfected cell line with at least one of a microorganism, a gene from a microorganism or a tumor gene, thereby providing an infected cell line;
culturing the uninfected cell line and the infected cell line under conditions which allow for expression of the recombinant individual soluble MHC class I allele from the construct, such conditions also allowing for endogenous loading of a peptide ligand into the antigen binding groove of each recombinant individual soluble MHC class I molecule allele in the presence of beta-2-microglobulin to form individual soluble MHC class I trimolecular complexes prior to secretion of the individual soluble MHC class I trimolecular complexes from the cell;
isolating the secreted individual soluble MHC class I trimolecular complexes from the uninfected cell line and the infected cell line;
separating the endogenously loaded peptide ligands from the individual soluble MHC class I trimolecular complexes from the uninfected cell line and separating the endogenously loaded peptide ligands from the individual soluble MHC class I trimolecular complexes from the infected cell line;
isolating the endogenously loaded peptide ligands from the uninfected cell line and the endogenously loaded peptide ligands from the infected cell line;
comparing the endogenously loaded peptide ligands isolated from the uninfected cell line to the endogenously loaded peptide ligands isolated from the infected cell line; and
identifying at least one endogenously loaded peptide ligand presented by the individual soluble MHC class I allele on the uninfected cell line that is not presented by the individual soluble MHC class I trimolecular complex on the infected cell line.

**16.** The method of claim 15 further comprising the step of identifying a source protein from which the at least one endogenously loaded peptide ligand presented by the individual soluble MHC class I trimolecular complex on the uninfected cell line and not presented by the individual soluble MHC class I trimolecular complex on the infected cell line is obtained.

**17.** The method of claim 15 wherein, in the step of infecting a portion of the uninfected cell line, the portion of the uninfected cell line is infected with HIV.

18. The method of claim 1 wherein, in the step of identifying at least one individual soluble MHC class I trimolecular complex, the trimolecular complex is identified using an antibody that recognizes the individual soluble MHC class I trimolecular complex having a peptide loaded therein.

**Patentansprüche**

1. Verfahren zum Assayieren eines Peptids zum Binden eines einzelnen, löslichen MHC-Klasse-I-Moleküls, welches Verfahren die Schritte umfasst:

   Bereitstellen eines zu untersuchenden Peptids;
   Bereitstellen eines Pools von einzelnen, löslichen MHC-Klasse-I-trimolekularen Komplexen, wobei jeder trimolekulare Komplex ein rekombinantes, lösliches MHC-Klasse-I-Allel aufweist, ein beta-2-Mikroglobulin und endogen geladenes Peptid, indem:

   eine Genom-DNA oder -cDNA erhalten wird, die mindestens ein MHC-Klasse-I-Molekül codiert;

   Identifizieren eines MHC-Klasse-I-Allels in der Genom-DNA oder -cDNA;
   PCR-Amplifizieren des MHC-Klasse-I-Allels in einer für den Locus spezifischen Weise,

   um ein PCR-Produkt zu erzeugen, welches eine abgestumpfte, lösliche Form des einzelnen MHC-Klasse-I-Allels codiert;

   Klonen des PCR-Produktes in einen Expressionsvektor, wodurch ein Konstrukt erzeugt wird, welches das einzelne, lösliche MHC-Klasse-I-Allel codiert;
   Transfizieren einer Säugerzelllinie, um eine Säugerzelllinie zu schaffen, die ein Konstrukt exprimiert, welches ein rekombinantes einzelnes, lösliches MHC-Klasse-I-Allel codiert, wobei die Säugerzelllinie in der Lage ist, Proteine auf natürliche Weise in Peptid-Liganden einzuarbeiten, um in Antigen-bindende Vertiefungen von MHC-Klasse-I-Molekülen geladen zu werden;
   Kultivieren der Säugerzelllinie unter Bedingungen, welche die Expression des rekombinanten, einzelnen MHC-Klasse-I-Allels aus dem Konstrukt erlauben, wobei diese Bedingungen auch ein endogenes Laden eines Peptid-Liganden in die Antigen-bindende Vertiefung jedes einzelnen, löslichen MHC-Klasse-I-Allel in Gegenwart von beta-2-Mikroglobulin ermöglicht, um die einzelnen, löslichen MHC-Klasse-I-trimolekularen Komplexe vor der Ausscheidung der einzelnen, löslichen MHC-Klasse-I-trimolekularen Komplexe aus der Zelle zu erzeugen; und
   Isolieren des Pools einzelner, löslicher MHC-Klasse-I-trimolekularer Komplexe, wobei ein solcher Schritt der Isolierung nicht zu einer selektiven Reinigung spezieller MHC-Klasse-I-trimolekularer Komplexe führt, die darin bestimmte Peptide geladen haben derart, dass der Pool einzelner, löslicher MHC-Klasse-I-trimolekularer Komplexe in den darin geladenen Peptiden nicht verzerrt wird;
   Mischen des zu untersuchenden Peptids mit den einzelnen, löslichen Klasse-I-trimolekularen Komplexen und
   Identifizieren mindestens eines einzelnen, löslichen MHC-Klasse-I-trimolekularen Komplexes, wobei das zu untersuchende Peptid ein endogen geladenes Peptid verdrängt hat und kompetitiv an dem löslichen MHC-Klasse-I-Allel und beta-2-Mikroglobulin gebunden ist, um den trimolekularen Komplex zu bilden.

2. Verfahren nach Anspruch 1, ferner umfassend den Schritt des Behandelns des Pools von einzelnen, löslichen MHC-Klasse-I-trimolekularen Komplexen unter Bedingungen, die ein Freisetzen der endogenen Peptide vor dem Mischen des zu untersuchenden Peptids mit dem Pool einzelner, löslicher MHC-Klasse-1-trimolekularer Komplexe bewirken.

3. Verfahren nach Anspruch 2, wobei der Schritt des Behandelns des Pools von einzelnen, löslichen MHC-Klasse-1-trimolekularen Komplexen das Erhitzen des Pools einzelner, löslicher MHC-Klasse-l-trimolekularer Komplexe umfasst, um die Freisetzung der endogenen Peptide zu bewirken.

4. Verfahren nach Anspruch 1, wobei in den Schritten des Bereitstellens des zu untersuchenden Peptids und des Identifizierens von mindestens einem einzelnen, löslichen MHC-Klasse-I-trimolekularen Komplex, worin das zu untersuchende Peptid ein endogen geladenes Peptid verdrängt hat, das zu untersuchende Peptid markiert ist, um eine Identifikation von mindestens einem einzelnen, löslichen MHC-Klasse-l-trimolekularen Komplex von dem ungebundenen, zu untersuchenden Peptid zu ermöglichen.

5. Verfahren nach Anspruch 4, wobei das zu untersuchende Peptid markiert ist mit einer radioaktiven Markierung oder mit einer Fluoreszenzmarkierung.

6. Verfahren nach Anspruch 5, wobei in dem Schritt des Identifizierens von mindestens einem einzelnen, löslichen MHC-Klasse-I-trimolekularen Komplex, worin das zu untersuchende Peptid ein endogen geladenes Peptid verdräng hat, das zu untersuchende Peptid markiert ist mit einer Fluoreszenzmarkierung und der mindestens eine einzelne, lösliche MHC-Klasse-I-trimolekulare Komplex mit Hilfe der Fluoreszenzpolarisation identifiziert wird.

7. Verfahren nach Anspruch 1, wobei das Konstrukt ferner einen Anhang codiert, das an dem einzelnen, löslichen MHC-Klasse-I-Allel angebracht ist und die Isolierung des Pools von einzelnen, löslichen MHC-Klasse-I-trimolekularen Komplexen unterstützt.

8. Verfahren nach Anspruch 7, wobei der Anhang ausgewählt ist aus der Gruppe, bestehend aus einem HIS-Schwanz und einem FLAG-Schwanz.

9. Verfahren nach Anspruch 1, wobei in dem Schritt der Bereitstellung eines zu untersuchenden Peptids das zu untersuchende Peptid identifiziert wird mit Hilfe einer Methoden zum Identifizieren mindestens eines endogen geladenen Peptid-Liganden, der eine infizierte Zelle von einer nichtinfizierten Zelle unterscheidet, welches Verfahren die Schritte umfasst:

Bereitstellen einer nichtinfizierten Zelllinie, die ein Konstrukt enthält, welches ein einzelnes, lösliches MHC-Klasse-I-Allel codiert, wobei die nichtinfizierte Zelllinie in der Lage ist, auf natürlichem Weg Proteine in Peptid-Liganden einzuarbeiten, die in der Lage sind, in Antigen-bindende Vertiefungen von MHC-Klasse-I-Molekülen geladen zu werden;
Infizieren eines Teil der nichtinfizierten Zelllinie mit mindestens einem Mikroorganismus, einem Gen aus einem Mikroorganismus oder einem Tumorgen, wodurch eine infizierte Zelllinie geschaffen wird;
Kultivieren der nichtinfizierten Zellinie und der infizierten Zellinie unter Bedingungen, die eine Expression des rekombinanten, einzelnen, löslichen MHC-Klasse-I-Allels aus dem Konstrukt ermöglichen, wobei diese Bedingungen ebenfalls ein endogenes Laden eines Peptid-Liganden in die Antigen-bindende Vertiefung jedes rekombinanten, einzelnen, löslichen MHC-Klasse-I-Allels in Gegenwart von beta-2-Miktroglobulin ermöglichen, um einzelne, lösliche MHC-Klasse-I-trimolekulare Komplexe vor der Ausscheidung der einzelnen, löslichen MHC-Klasse-I-trimolekularen Komplexe aus der Zelle zu erzeugen;
Isolieren der ausgeschiedenen, einzelnen, löslichen MHC-Klasse-I-trimolekularen Komplexe aus der nichtinfizierten Zelllinie und der infizierten Zellinie ;
Abtrennen der endogen geladenen Peptid-Liganden aus den einzelnen, löslichen MHC-Klasse-I-trimolekularen Komplexen aus der nichtinfizierten Zelllinie und Abtrennen der endogen geladenen Peptid-Liganden aus den einzelnen, löslichen MHC-Klasse-I-trimolekularen Komplexen aus der infizierten Zelllinie;
Isolieren der endogen geladenen Peptid-Liganden aus der nichtinfizierten Zelllinie und den endogen geladenen Peptid-Liganden aus der infizierten Zelllinie;
Vergleichen der endogen geladenen Peptid-Liganden, die aus der infizierten Zelllinie isoliert wurden, mit den endogen geladenen Peptid-Liganden, die aus der nichtinfizierten Zelllinie isoliert wurden; sowie
Identifizieren von mindestens einem endogen geladenen Peptid-Liganden, der auf dem einzelnen, löslichen MHC-Klasse-I-Allel auf der infizierten Zelllinie präsentiert wird, der nichtpräsentiert wird von dem einzelnen, löslichen MHC-Klasse-I-Allel auf der nichtinfizierten Zelllinie.

10. Verfahren nach Anspruche 9, ferner umfassend den Schritt des Identifizierens eines Quellproteins, aus dem der mindestens eine endogen geladene Peptid-Ligand, der von dem einzelnen, löslichen MHC-Klasse-I-trimolekularen Komplex auf der infizierten Zelllinie präsentiert wird und nicht präsentiert wird durch den einzelnen, löslichen MHC-Klasse-I-trimolekularen Komplex auf der nichtinfizierten Zelllinie, erhalten wird.

11. Verfahren nach Anspruch 9, wobei in dem Schritt des Identifizierens von mindestens einem endogen geladenen Peptid-Liganden, der von dem einzelnen, löslichen MHC-Klasse-I-trimolekularen Komplex auf der infizierten Zelllinie präsentiert wird, jedoch nicht auf der nichtinfizierten Zelllinie präsentiert wird, der mindestens eine endogen geladene Peptid-Ligand erhalten wird von einem Protein, das codiert wird von mindestens einem der Mikroorganismen, dem Gen von einem Mikroorganismus oder dem Tumorgen, mit dem die Zelllinie infiziert wurde, um die infizierte Zelllinie zu erzeugen.

12. Verfahren nach Anspruch 9, wobei in dem Schritt des Identifizierens von mindestens einem endogen geladenen Peptid-Liganden, der von dem einzelnen, löslichen MHC-Klasse-I-trimolekularen Komplex auf der infizierten Zelllinie präsentiert wird, der nicht jedoch auf der nichtinfizierten Zelllinie präsentiert wird, der mindestens eine endogen geladene Peptid-Ligand erhalten wird aus einem Protein, das durch die nichtinfizierte Zelllinie codiert wird.

**13.** Verfahren nach Anspruch 12, wobei das Protein, das durch die nichtinfizierte Zelllinie codiert wird, von der der mindestens eine endogen geladene Peptid-Ligand erhalten wird, über eine erhöhte Expression in einer Tumorzelllinie verfügt.

**14.** Verfahren nach Anspruch 9, wobei in dem Schritt des Infizierens eines Teils der nichtinfizierten Zelllinie der Teil der nichtinfizierten Zelllinie mit HIV infiziert wird.

**15.** Verfahren nach Anspruch 1, wobei in dem Schritt der Bereitstellung eines zu untersuchenden Peptids das zu untersuchende Peptid mit Hilfe einer Methode zum Identifizieren von mindestens einem endogen geladenen Peptid-Liganden identifiziert wird, der eine infizierte Zelle von einer nichtinfizierten Zelle unterscheidet, welches Verfahren die Schritte umfasst:

Bereitstellen einer nichtinfizierten Zelllinie, die ein Konstrukt enthält, das ein einzelnes, lösliches MHC-Klasse-I-Allel codiert, wobei die nichtinfizierte Zelllinie in der Lage ist, auf natürliche Weise Proteine in Peptid-Liganden einzuarbeiten, die in Antigen-bindende Vertiefungen von MHC-Klasse-I-Molekülen geladen werden können;
Infizieren eines Teils der nichtinfizierten Zelllinie mit mindestens einem Mikroorganismus, einem Gen aus einem Mikroorganismus oder einem Tumorgen, wodurch eine infizierte Zelllinie geschaffen wird;
Kultivieren der nichtinfizierten Zelllinie und der infizierten Zelllinie unter Bedingungen, die die Expression des rekombinanten, einzelnen, löslichen MHC-Klasse-I-Allels aus dem Konstrukt ermöglichen, wobei diese Bedingungen ebenfalls ein endogenes Laden eines Peptid-Liganden in die Antigen-bindende Vertiefung jedes rekombinanten, einzelnen, löslichen MHC-Klasse-I-Molekül-Allels in Gegenwart von beta-2-Mikroglobulin ermöglichen, um einzelne, lösliche MHC-Klasse-I-trimolekulare Komplexe vor der Ausscheidung der einzelnen, löslichen MHC-Klasse-I-trimolekularen Komplexe aus der Zelle zu erzeugen;
Isolieren der ausgeschiedenen, einzelnen, löslichen MHC-Klasse-I-trimolekularen Komplexe aus der nichtinfizierten Zelllinie und der infizierten Zelllinie;
Abtrennen der endogen geladenen Peptid-Liganden aus den einzelnen, löslichen MHC-Klasse-I-trimolekularen Komplexen aus der nichtinfizierten Zelllinie und Abtrennen der endogen geladenen Peptid-Liganden von den einzelnen, löslichen MHC-Klasse-I-trimolekukaren Komplexen aus der infizierten Zelllinie;
Isolieren der endogen geladenen Peptid-Liganden aus der nichtinfizierten Zelllinie und der endogen geladenen Peptid-Liganden aus der infizierten Zelllinie;
Vergleichen der endogen geladenen Peptid-Liganden, die aus der nichtinfizierten Zelllinie isoliert wurden, mit den endogen geladenen Peptid-Liganden, die aus der infizierten Zelllinie isoliert wurden; sowie
Idendifizieren von mindestens einem endogen geladenen Peptid-Liganden, der von dem einzelnen, löslichen MHC-Klasse-I-Allel auf der nichtinfizierten Zelllinie präsentiert wird, der nicht von dem einzelnen, löslichen MHC-Klasse-I-trimolekularen Komplex auf der infizierten Zelllinie präsentiert wird.

**16.** Verfahren nach Anspruch 15, ferner umfassend den Schritt des Identifizierens eines Quellproteins, aus dem mindestens ein endogen geladener Peptid-Ligand, der von dem einzelnen, löslichen MHC-Klasse-I-trimolekularen Komplex auf der nichtinfizierten Zelllinie präsentiert wird und nicht von dem einzelnen, löslichen MHC-Klasse-I-trimolekularen Komplex auf der infizierten Zelllinie präsentiert wird, erhalten wird.

**17.** Verfahren nach Anspruch 15, wobei in dem Schritt des Infizierens eines Teils der nichtinfizierten Zelllinie der Teil der nichtinfizierten Zelllinie mit HIV infiziert wird.

**18.** Verfahren nach Anspruch 1, wobei in dem Schritt des Identifizierens von mindestens einem einzelnen, löslichen MHC-Klasse-I-trimolekularen Komplex der trimolekulare Komplex identifiziert wird, indem ein Antikörper verwendet wird, welcher den einzelnen, löslichen MHC-Klasse-I-trimolekularen Komplex mit einem darin geladenen Peptid erkennt.

## Revendications

**1.** Procédé pour tester la liaison d' un peptide à une molécule de CMH de classe I individuelle soluble, comprenant les étapes suivantes :

- on prépare un peptide intéressant ;
- on prépare un ensemble de complexes trimoléculaires individuels solubles de CMH de classe I, chaque complexe trimoléculaire comprenant un allèle de CMH de classe I recombinant soluble, une bêta-2-microglobuline et un peptide chargé par voie endogène, au moyen des étapes suivantes :

  • on se procure de l'ADN génomique ou de l'ADNc génomique codant pour au moins une molécule de CMH de classe I ;
  • on identifie un allèle de CMH de classe I dans l'ADN génomique ou dans l'ADNc génomique ;
  • on amplifie par PCR l'allèle de CMH de classe I d'une manière spécifique au locus, afin d'obtenir un produit de PCR qui code pour une forme tronquée soluble de l'allèle de CMH de classe I individuel ;
  • on clone le produit de PCR dans un vecteur d'expression, ce qui donne ainsi une construction qui code pour l'allèle de CMH de classe I individuel soluble ;
  • on transfecte une lignée cellulaire mammifère pour obtenir une lignée cellulaire mammifère exprimant une construction qui code pour un allèle de CMH de classe I recombinant individuel soluble, la lignée cellulaire mammifère étant capable d'effectuer l'apprêtement naturel des protéines sous forme de ligands peptidiques destinés à être chargés dans les sillons de liaison aux antigènes des molécules de CMH de classe I ;
  • on met en culture la lignée cellulaire mammifère dans des conditions qui permettent l'expression de l'allèle de CMH de classe I recombinant individuel à partir de la construction, les conditions en question permettant également qu'un ligand peptidique soit chargé dans le sillon de liaison aux antigènes de chaque allèle de CMH de classe I individuel soluble en présence de bêta-2-microglobuline, dans le but de former les complexes trimoléculaires individuels solubles de CMH de classe I avant la sécrétion des complexes trimoléculaires individuels solubles de CMH de classe I par la cellule ; et
  • on isole l'ensemble des complexes trimoléculaires individuels solubles de CMH de classe I, cette étape d'isolement n'entraînant pas de purification sélective de complexes trimoléculaires particuliers de CMH de classe I sur lesquels certains peptides sont chargés, de sorte que l'ensemble des complexes trimoléculaires individuels solubles de CMH de classe I ne soit pas biaisé en faveur des peptides chargés sur ceux-ci ;

- on mélange le peptide intéressant aux complexes trimoléculaires individuels solubles de classe I ; et
- on identifie au moins un complexe trimoléculaire individuel soluble de CMH de classe I, dans lequel le peptide d'intérêt a déplacé un peptide chargé par voie endogène et se lie par compétition à l'allèle de CMH de classe I soluble et à la bêta-2-microglobuline pour former le complexe trimoléculaire.

**2.** Procédé selon la revendication 1, comprenant en outre l'étape consistant à traiter l'ensemble des complexes trimoléculaires individuels solubles de CMH de classe I dans des conditions qui provoquent la libération des peptides endogènes avant de mélanger le peptide intéressant à l'ensemble des complexes trimoléculaires individuels solubles de CMH de classe I.

**3.** Procédé selon la revendication 2, dans lequel l'étape de traitement de l'ensemble des complexes trimoléculaires individuels solubles de CMH de classe I implique de chauffer l'ensemble des complexes trimoléculaires individuels solubles de CMH de classe I pour provoquer la libération des peptides endogènes.

**4.** Procédé selon la revendication 1 dans lequel, aux étapes de préparation du peptide intéressant et d'identification d'au moins un complexe trimoléculaire individuel soluble de CMH de classe I, étape à laquelle le peptide intéressant a déplacé un peptide chargé par voie endogène, le peptide intéressant est marqué afin de pouvoir identifier le au moins un complexe trimoléculaire individuel soluble de CMH de classe I du peptide intéressant non lié.

**5.** Procédé selon la revendication 4, dans lequel le peptide intéressant est marqué avec un marqueur radioactif ou avec un marqueur fluorescent.

**6.** Procédé selon la revendication 5 dans lequel, à l'étape d'identification d'au moins un complexe trimoléculaire individuel soluble de CMH de classe I, étape à laquelle le peptide intéressant a déplacé un peptide chargé par voie endogène, le peptide intéressant est marqué avec un marqueur fluorescent et on identifie le au moins un complexe trimoléculaire individuel soluble de CMH de classe I par polarisation de fluorescence.

**7.** Procédé selon la revendication 1, dans lequel la construction code également pour une étiquette qui est fixée à l'allèle de CMH de classe I individuel soluble et qui facilite la phase d'isolement de l'ensemble des complexes trimoléculaires individuels solubles de CMH de classe I.

**8.** Procédé selon la revendication 7, dans lequel l'étiquette est choisie dans le groupe constitué d'une séquence de queue de type HIS et d'une séquence de queue de type FLAG.

**9.** Procédé selon la revendication 1 dans lequel, à l'étape de préparation d'un peptide intéressant, on identifie le peptide intéressant au moyen d'un procédé consistant à identifier au moins un ligand peptidique chargé par voie endogène qui discrimine une cellule infectée d'une cellule non infectée, le procédé comprenant les étapes suivantes :

- on prépare une lignée cellulaire non infectée contenant une construction qui code pour un allèle de CMH de classe I individuel soluble, la lignée cellulaire non infectée étant capable d'effectuer l'apprêtement naturel des protéines sous forme de ligands peptidiques pouvant être chargés dans les sillons de liaison aux antigènes de molécules de CMH de classe I ;
- on infecte une partie de la lignée cellulaire non infectée avec au moins un micro-organisme, un gène provenant d'un micro-organisme ou un gène tumoral, ce qui donne une lignée cellulaire infectée ;
- on met en culture la lignée cellulaire non infectée et la lignée cellulaire infectée dans des conditions qui permettent l'expression de l'allèle de CMH de classe I recombinant individuel soluble à partir de la construction, ces conditions permettant également qu'un ligand peptidique soit chargé par voie endogène dans le sillon de liaison aux antigènes de chaque allèle de CMH de classe I recombinant individuel soluble en présence de bêta-2-microglobuline, dans le but de former des complexes trimoléculaires individuels solubles de CMH de classe I avant la sécrétion des complexes trimoléculaires individuels solubles de CMH de classe I par la cellule ;
- on isole les complexes trimoléculaires individuels solubles de CMH de classe I secrétés, à partir de la lignée cellulaire non infectée et à partir de la lignée cellulaire infectée ;
- on sépare les ligands peptidiques chargés par voie endogène des complexes trimoléculaires individuels solubles de CMH de classe I à partir de la lignée cellulaire non infectée et on sépare les ligands peptidiques chargés par voie endogène des complexes trimoléculaires individuels solubles de CMH de classe I à partir de la lignée cellulaire infectée ;
- on isole les ligands peptidiques chargés par voie endogène à partir de la lignée cellulaire non infectée et les ligands peptidiques chargés par voie endogène à partir de la lignée cellulaire infectée ;
- on compare les ligands peptidiques chargés par voie endogène isolés à partir de la lignée cellulaire infectée aux ligands peptidiques chargés par voie endogène isolés à partir de la lignée cellulaire non infectée ; et
- on identifie au moins un ligand peptidique chargé par voie endogène présenté par l'allèle de CMH de classe I individuel soluble dans la lignée cellulaire infectée, qui n'est pas présenté par l'allèle de CMH de classe I individuel soluble dans la lignée cellulaire non infectée.

**10.** Procédé selon la revendication 9, comprenant encore l'étape d'identification d'une protéine source à partir de laquelle on obtient le au moins un ligand peptidique chargé par voie endogène qui est présenté par le complexe trimoléculaire individuel soluble de CMH de classe I dans la lignée cellulaire infectée et qui n'est pas présenté par le complexe trimoléculaire individuel soluble de CMH de classe I dans la lignée cellulaire non infectée.

**11.** Procédé selon la revendication 9 dans lequel, à l'étape d'identification d'au moins un ligand peptidique chargé par voie endogène présenté par le complexe trimoléculaire individuel soluble de CMH de classe I dans la lignée cellulaire infectée, mais qui n'est pas présenté dans la lignée cellulaire non infectée, on obtient le au moins un ligand peptidique chargé par voie endogène à partir d'une protéine codée selon au moins l'une des approches impliquant le micro-organisme, le gène d'un micro-organisme ou le gène tumoral, avec lequel la lignée cellulaire a été infectée pour donner la lignée cellulaire infectée.

**12.** Procédé selon la revendication 9 dans lequel, à l'étape d'identification d'au moins un ligand peptidique chargé par voie endogène et présenté par le complexe trimoléculaire individuel soluble de CMH de classe I dans la lignée cellulaire infectée, mais qui n'est pas présenté dans la lignée cellulaire non infectée, on obtient le au moins un ligand peptidique chargé par voie endogène à partir d'une protéine codée par la lignée cellulaire non infectée.

**13.** Procédé selon la revendication 12, dans lequel la protéine codée par la lignée cellulaire non infectée à partir de laquelle on obtient le au moins un ligand peptique chargé par voie endogène, présente une expression accrue dans une lignée cellulaire tumorale.

**14.** Procédé selon la revendication 9 dans lequel, à l'étape d'infection d'une partie de la lignée cellulaire non infectée, on infecte la partie de la lignée cellulaire non infectée avec le VIH.

**15.** Procédé selon la revendication 1 dans lequel, à l'étape de préparation d'un peptide intéressant, on identifie le peptide intéressant au moyen d'un procédé visant à identifier au moins un ligand peptidique chargé par voie endogène, qui discrimine une cellule infectée d'une cellule non infectée, le procédé comprenant les étapes suivantes :

- on prépare une lignée cellulaire non infectée contenant une construction qui code pour un allèle de CMH de classe I individuel soluble, la lignée cellulaire non infectée étant capable d'effectuer l'apprêtement naturel des protéines sous forme de ligands peptidiques pouvant être chargés dans les sillons de liaison aux antigènes de molécules de CMH de classe I ;
- on infecte une partie de la lignée cellulaire non infectée avec au moins un micro-organisme, un gène d'un micro-organisme ou un gène tumoral, ce qui donne une lignée cellulaire infectée ;
- on met en culture la lignée cellulaire non infectée et la lignée cellulaire infectée dans des conditions qui permettent l'expression de l'allèle de CMH de classe I recombinant individuel soluble à partir de la construction, ces conditions permettant également qu'un ligand peptidique soit chargé dans le sillon de liaison aux antigènes de chaque allèle de CMH de classe I recombinant individuel soluble en présence de bêta-2-microglobuline, dans le but de former des complexes trimoléculaires individuels solubles de CMH de classe I avant la sécrétion des complexes trimoléculaires individuels solubles de CMH de classe I par la cellule ;
- on isole les complexes trimoléculaires individuels solubles de CMH de classe I sécrétés, à partir de la lignée cellulaire non infectée et à partir de la lignée cellulaire infectée ;
- on sépare les ligands peptidiques chargés par voie endogène des complexes trimoléculaires individuels solubles de CMH de classe I à partir de la lignée cellulaire non infectée et on sépare les ligands peptidiques chargés par voie endogène des complexes trimoléculaires individuels solubles de CMH de classe I à partir de la lignée cellulaire infectée ;
- on isole les ligands peptidiques chargés par voie endogène à partir de la lignée cellulaire non infectée et les ligands peptidiques chargés par voie endogène à partir de la lignée cellulaire infectée ;
- on compare les ligands peptidiques chargés par voie endogène isolés de la lignée cellulaire non infectée aux ligands peptidiques chargés par voie endogène isolés de la lignée cellulaire infectée ; et
- on identifie au moins un ligand peptidique chargé par voie endogène présenté par l'allèle de CMH de classe I individuel soluble dans la lignée cellulaire non infectée, qui n'est pas présenté par l'allèle de CMH de classe I individuel soluble dans la lignée cellulaire infectée.

**16.** Procédé selon la revendication 15, comprenant également l'étape d'identification d'une protéine source à partir de laquelle on obtient le au moins un ligand peptidique chargé par voie endogène et présenté par le complexe trimoléculaire individuel soluble de CMH de classe I dans la lignée cellulaire non infectée, et qui n'est pas présenté par le complexe trimoléculaire individuel soluble de CMH de classe I dans la lignée cellulaire infectée.

**17.** Procédé selon la revendication 15 dans lequel, à l'étape d'infection d'une partie de la lignée cellulaire non infectée, on infecte la partie de la lignée cellulaire non infectée avec le VIH.

**18.** Procédé selon la revendication 1 dans lequel, à l'étape d'identification d'au moins un complexe trimoléculaire individuel soluble de CMH de classe I, on identifie le complexe trimoléculaire en utilisant un anticorps qui reconnaît le complexe trimoléculaire individuel soluble de CMH de classe I sur lequel un peptide est chargé.

Fig 1.

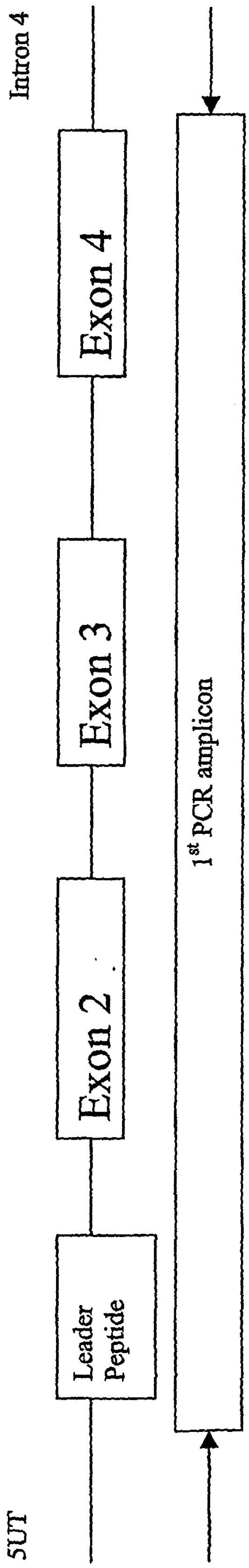
5UT
Leader Peptide
Exon 2
Exon 3
Exon 4
Intron 4
1st PCR amplicon

# FIGURE 2

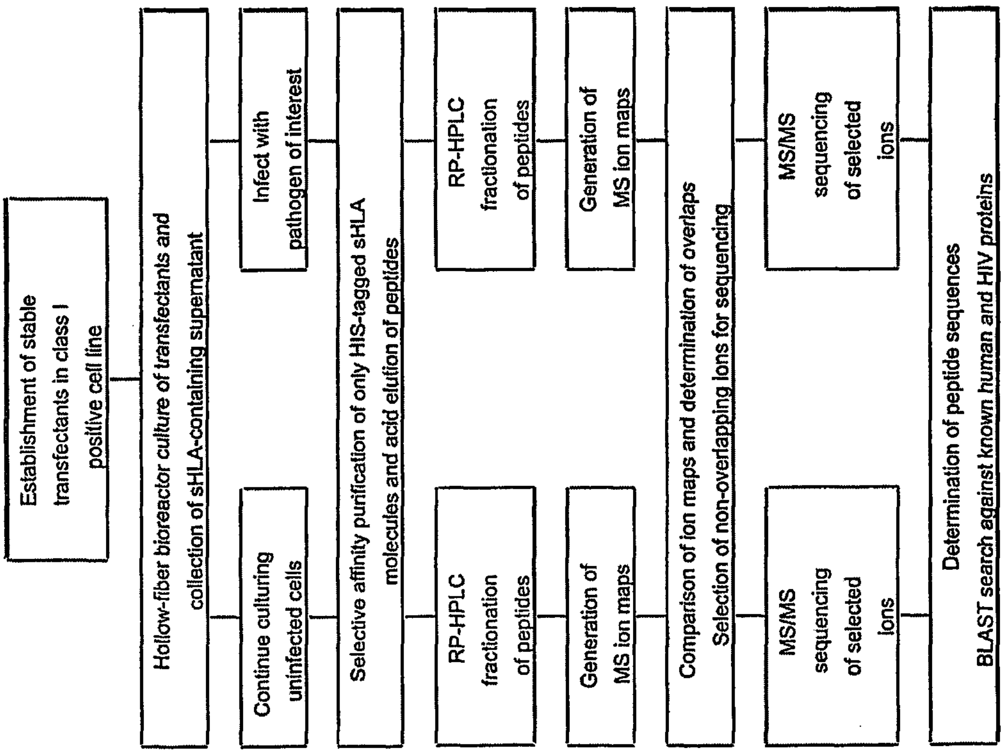
Establishment of stable transfectants in class I positive cell line
Hollow-fiber bioreactor culture of transfectants and collection of sHLA-containing supernatant
Continue culturing uninfected cells
Infect with pathogen of interest
Selective affinity purification of only HIS-tagged sHLA molecules and acid elution of peptides
RP-HPLC fractionation of peptides
RP-HPLC fractionation of peptides
Generation of MS ion maps
Generation of MS ion maps
Comparison of ion maps and determination of overlaps
Selection of non-overlapping ions for sequencing
MS/MS sequencing of selected ions
MS/MS sequencing of selected ions
Determination of peptide sequences
BLAST search against known human and HIV proteins

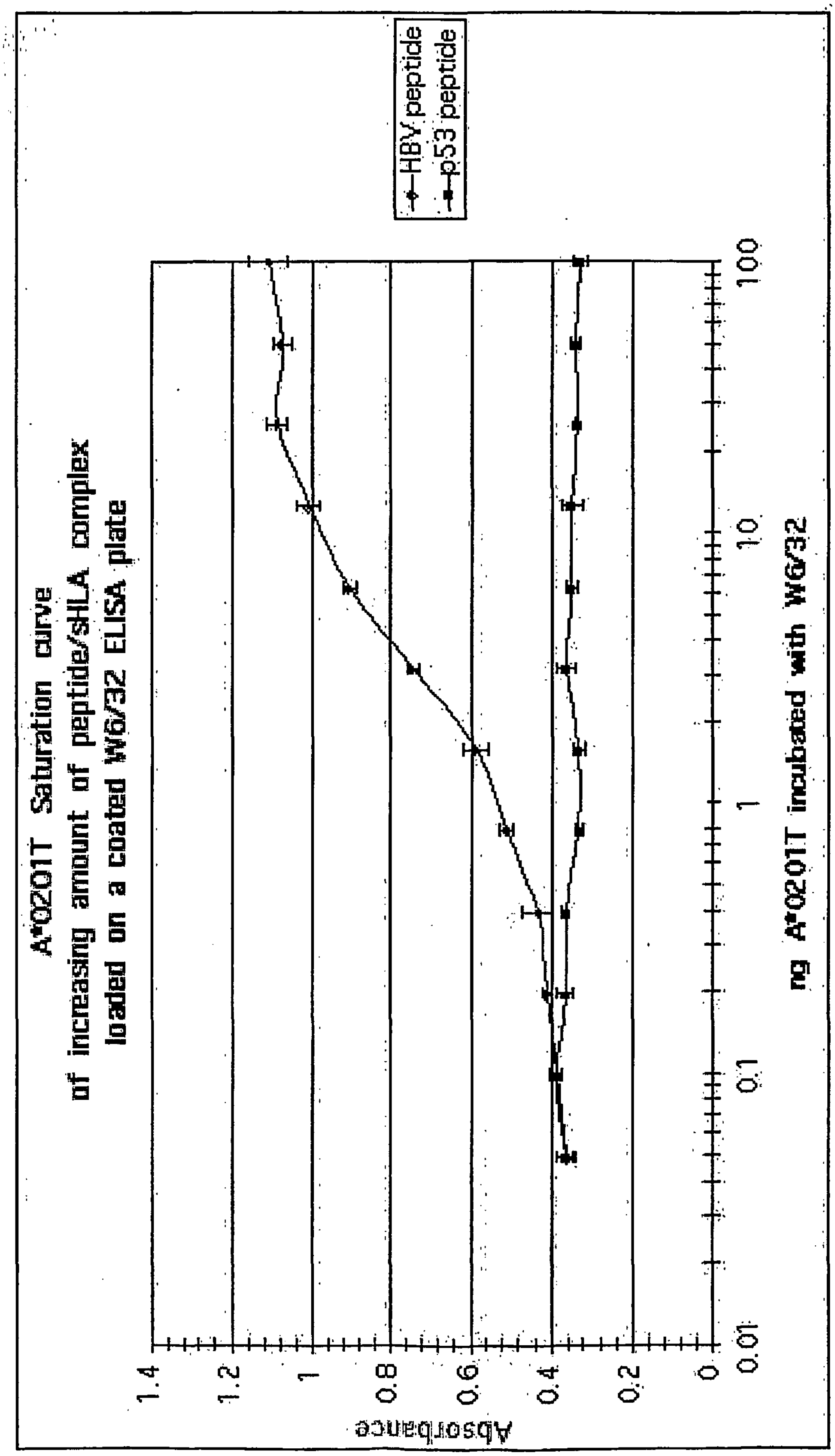
A*0201T Saturation curve
of increasing amount of peptide/sHLA complex
loaded on a coated W6/32 ELISA plate
HBV peptide
p53 peptide
Absorbance
1.4
1.2
1
0.8
0.6
0.4
0.2
0
0.01
0.1
1
10
100
ng A*0201T incubated with W6/32

**FIG. 3**

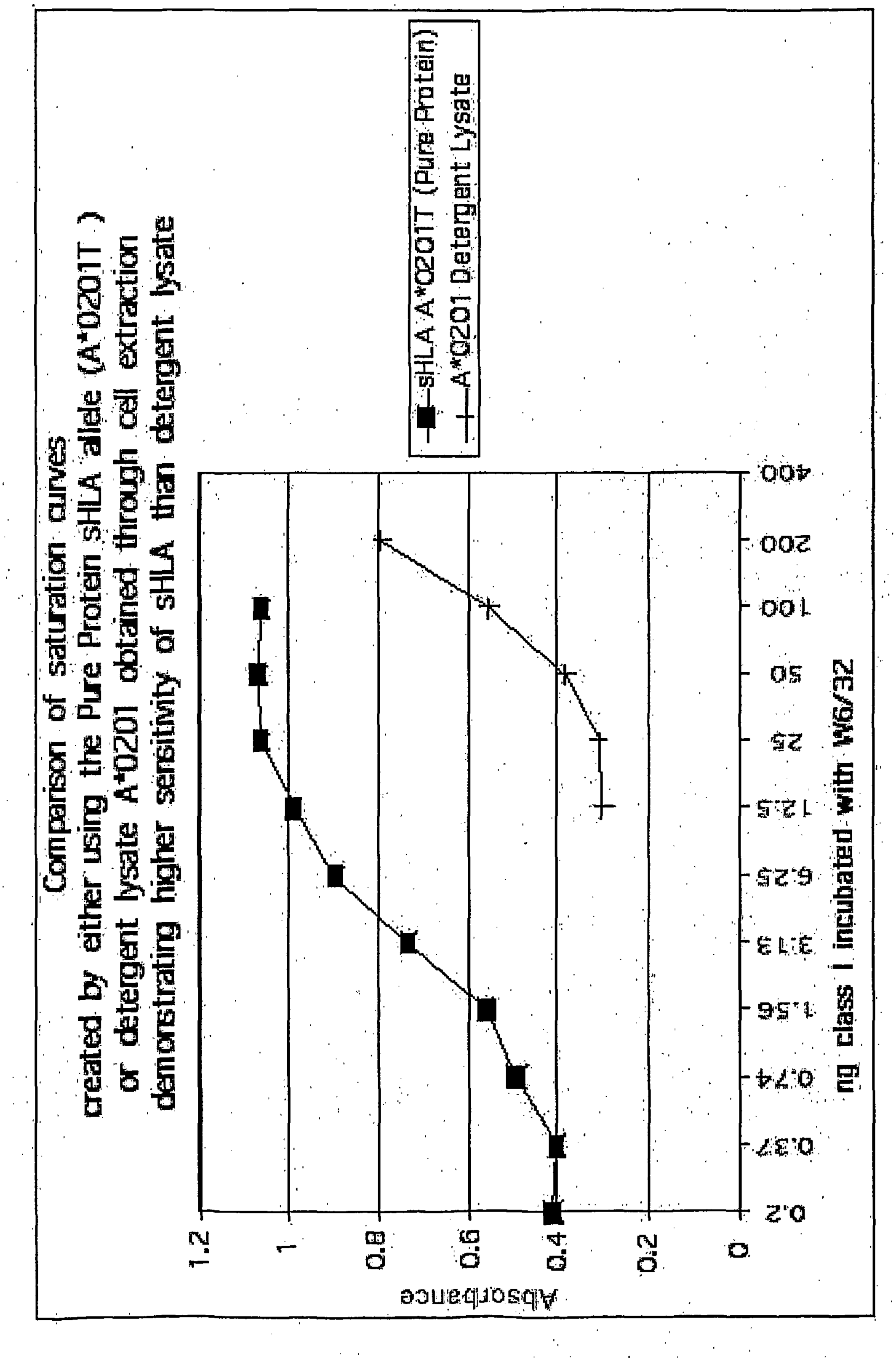
Comparison of saturation curves
created by either using the Pure Protein sHLA allele (A*0201T )
or detergent lysate A*0201 obtained through cell extraction
demonstrating higher sensitivity of sHLA than detergent lysate
sHLA A*0201T (Pure Protein)
A*0201 Detergent Lysate
Absorbance
1.2
1
0.8
0.6
0.4
0.2
0
0.2
0.37
0.74
1.56
3.13
6.25
12.5
25
50
100
200
400
ng class I incubated with W6/32

FIG. 4

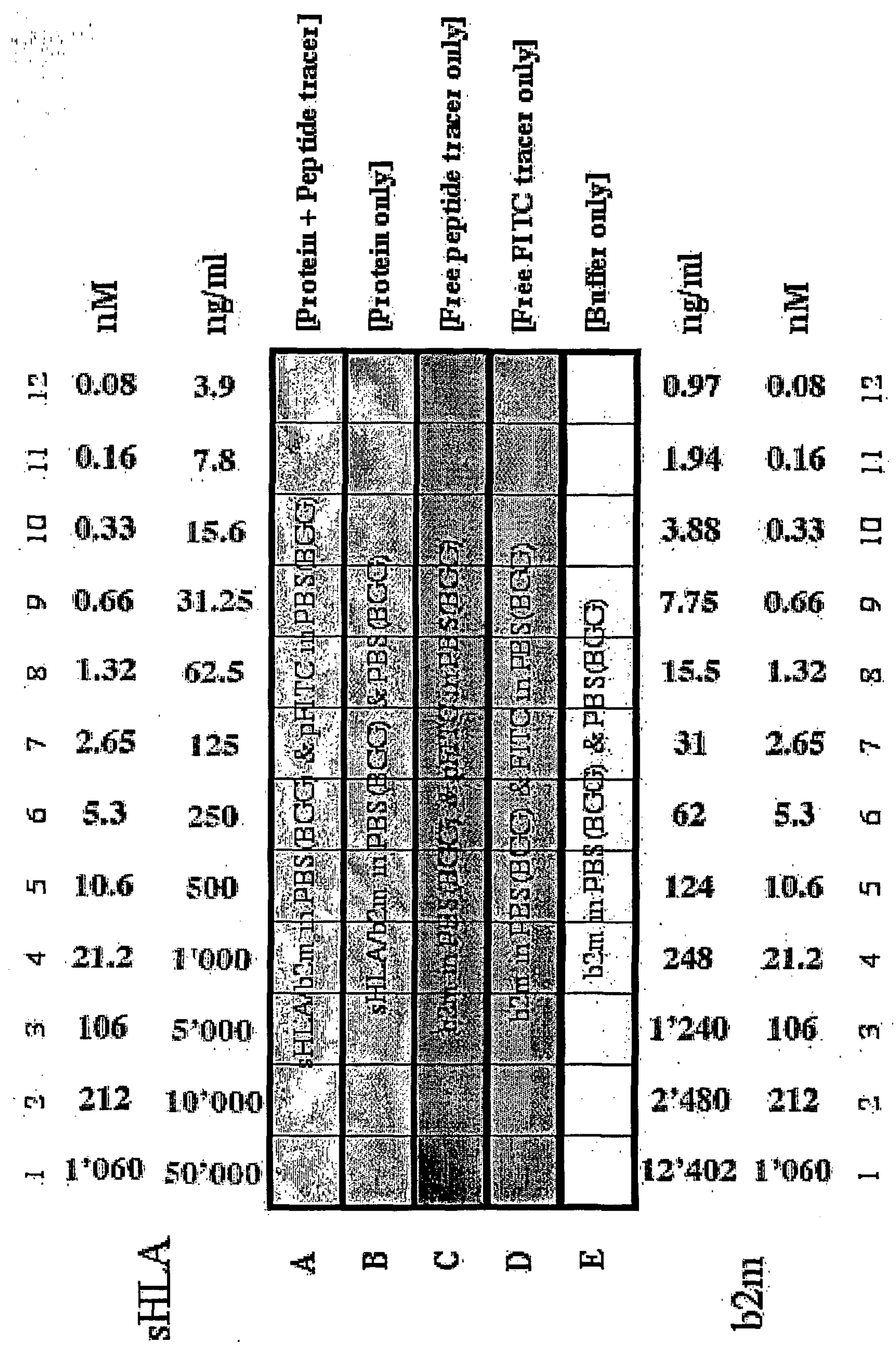
sHLA
nM
ng/ml
[Protein + Peptide tracer]
[Protein only]
[Free peptide tracer only]
[Free FITC tracer only]
[Buffer only]
ng/ml
nM
b2m
A
B
C
D
E
1 2 3 4 5 6 7 8 9 10 11 12
1'060 212 106 21.2 10.6 5.3 2.65 1.32 0.66 0.33 0.16 0.08
50'000 10'000 5'000 1'000 500 250 125 62.5 31.25 15.6 7.8 3.9
sHLA/b2m in PBS(BGG) & pFITC in PBS(BGG)
sHLA/b2m in PBS(BGG) & PBS(BGG)
b2m in PBS(BGG) & pFITC in PBS(BGG)
b2m in PBS(BGG) & FITC in PBS(BGG)
b2m in PBS(BGG) & PBS(BGG)
12'402 2'480 1'240 248 124 62 31 15.5 7.75 3.88 1.94 0.97
1'060 212 106 21.2 10.6 5.3 2.65 1.32 0.66 0.33 0.16 0.08

FIG. 5

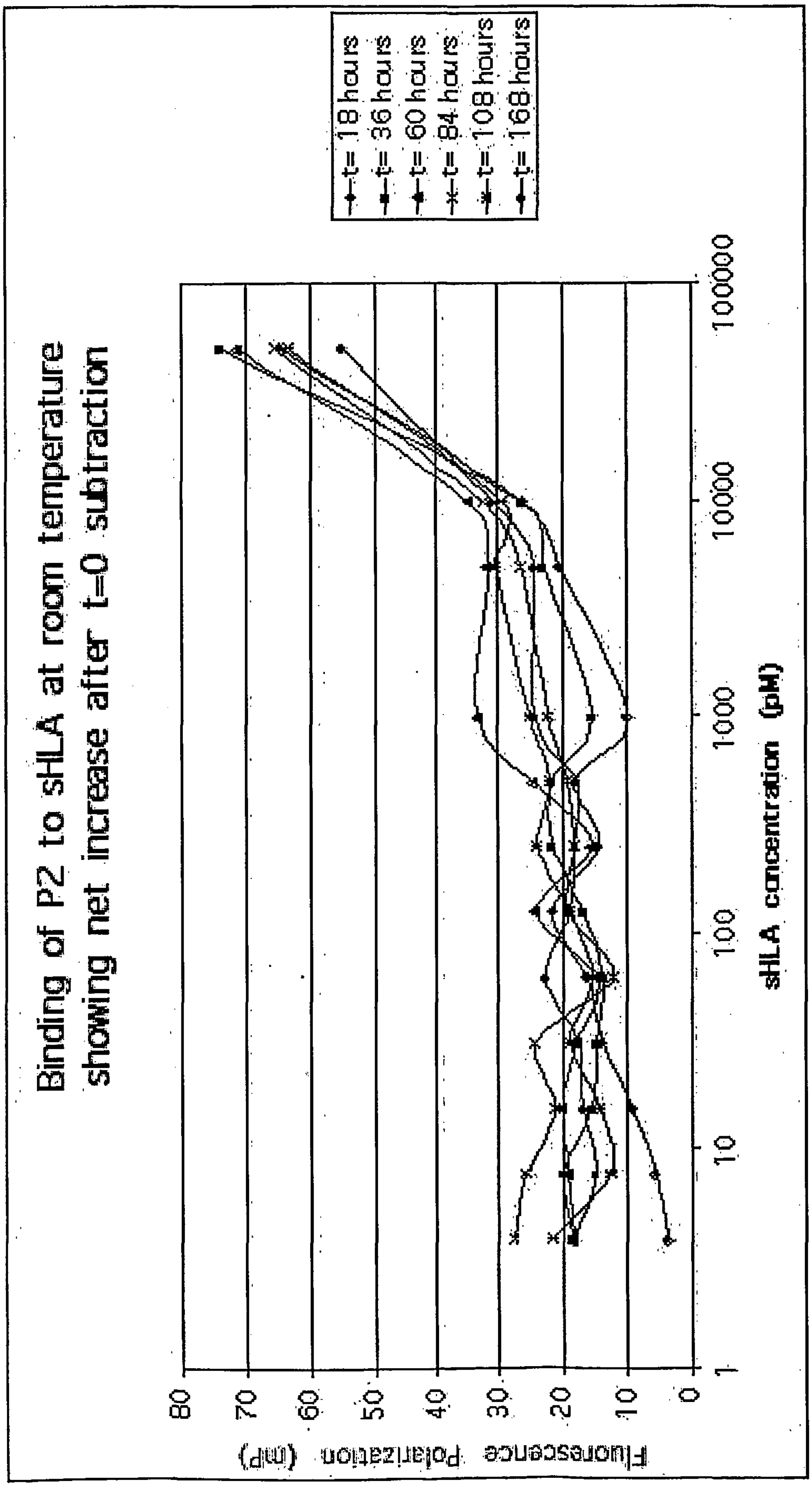

Binding of P2 to sHLA at room temperature
showing net increase after t=0 subtraction
t= 18 hours
t= 36 hours
t= 60 hours
t= 84 hours
t= 108 hours
t= 168 hours
Fluorescence Polarization (mP)
80
70
60
50
40
30
20
10
0
1
10
100
1000
10000
100000
sHLA concentration (pM)


FIG. 6

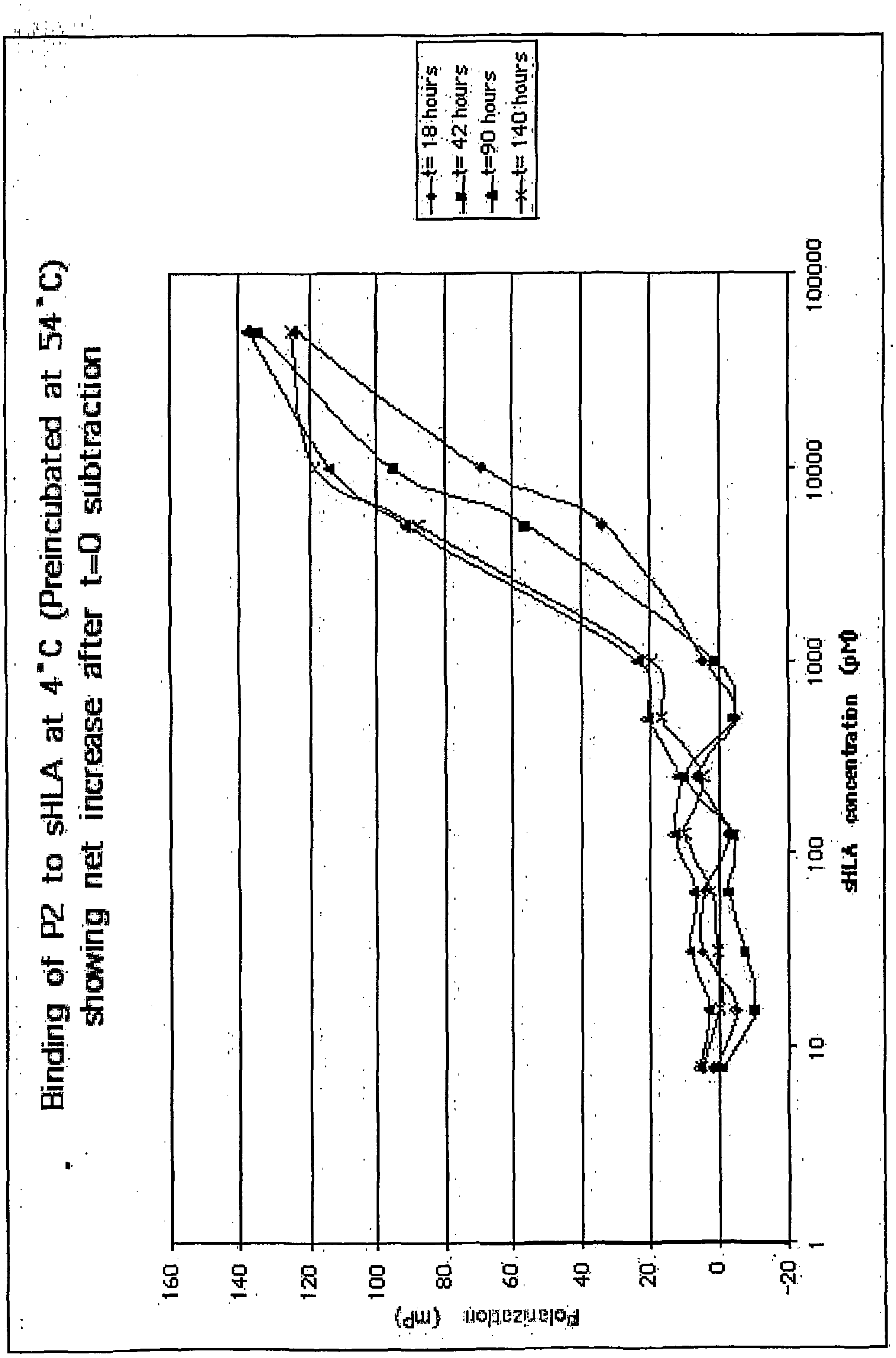
Binding of P2 to sHLA at 4°C (Preincubated at 54°C)
showing net increase after t=0 subtraction
t= 18 hours
t= 42 hours
t=90 hours
t= 140 hours
Polarization (mP)
160
140
120
100
80
60
40
20
0
-20
1
10
100
1000
10000
100000
sHLA concentration (pM)

FIG. 7

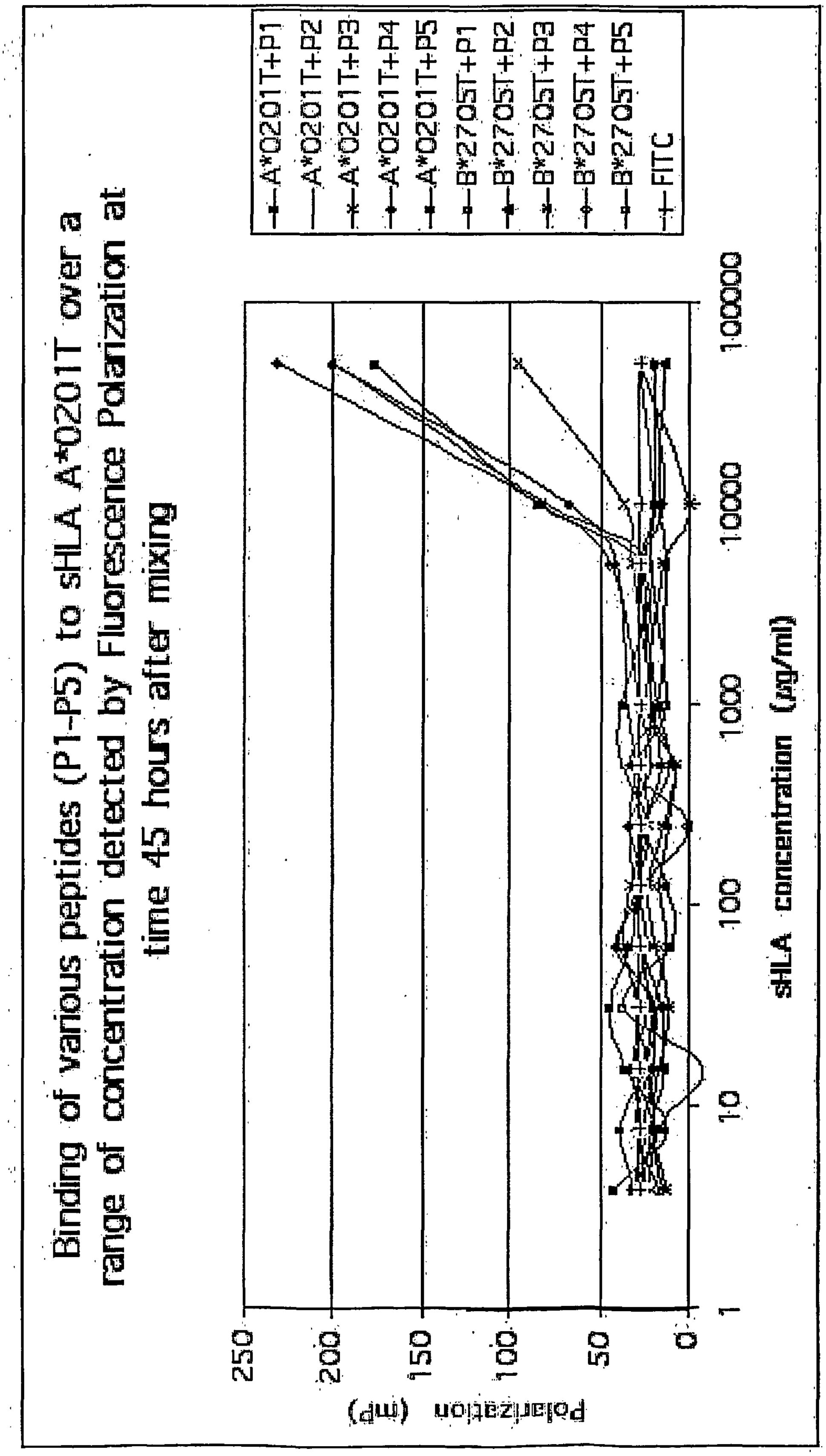
Binding of various peptides (P1-P5) to sHLA A*0201T over a range of concentration detected by Fluorescence Polarization at time 45 hours after mixing
A*0201T+P1
A*0201T+P2
A*0201T+P3
A*0201T+P4
A*0201T+P5
B*2705T+P1
B*2705T+P2
B*2705T+P3
B*2705T+P4
B*2705T+P5
FITC
Polarization (mP)
250
200
150
100
50
0
1
10
100
1000
10000
100000
sHLA concentration (µg/ml)

FIG. 8

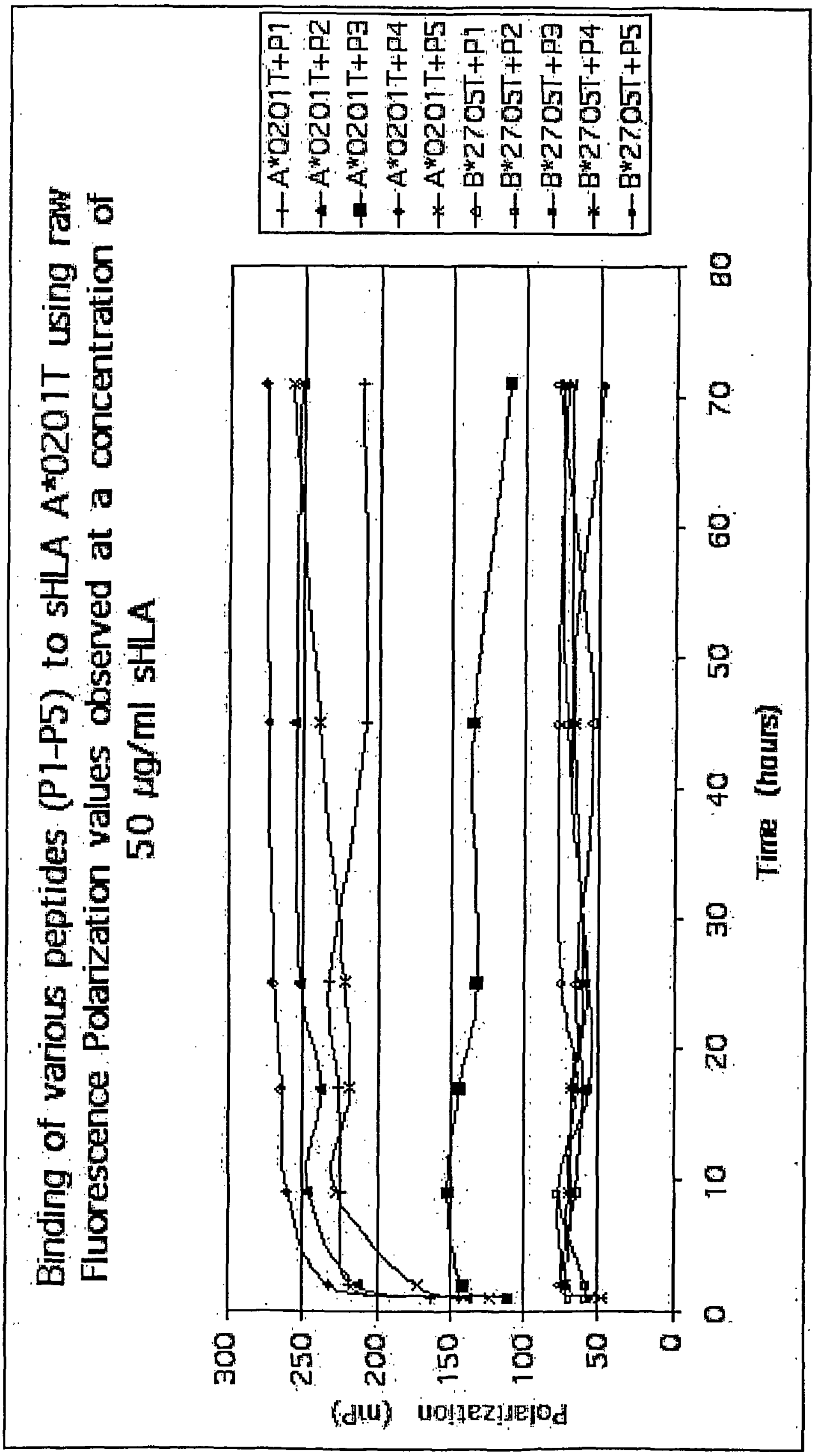
Binding of various peptides (P1-P5) to sHLA A*0201T using raw Fluorescence Polarization values observed at a concentration of 50 µg/ml sHLA
Polarization (mP)
300
250
200
150
100
50
0
0
10
20
30
40
50
60
70
80
Time (hours)
A*0201T+P1
A*0201T+P2
A*0201T+P3
A*0201T+P4
A*0201T+P5
B*2705T+P1
B*2705T+P2
B*2705T+P3
B*2705T+P4
B*2705T+P5

FIG. 9

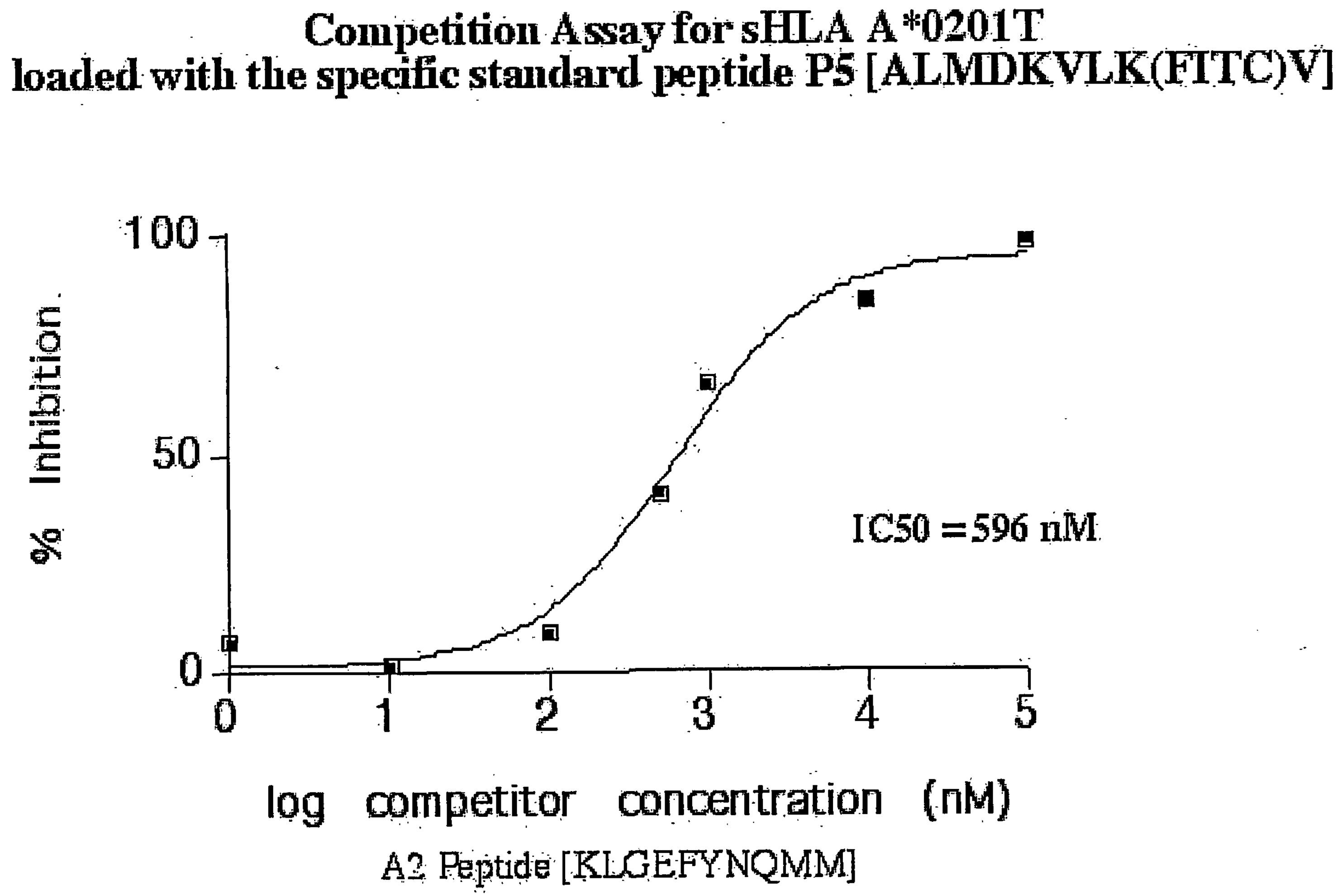
Competition Assay for sHLA A*0201T
loaded with the specific standard peptide P5 [ALMDKVLK(FITC)V]
100
50
0
% Inhibition
0
1
2
3
4
5
IC50 = 596 nM
log competitor concentration (nM)
A2 Peptide [KLGEFYNQMM]

**FIG. 10**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **COX et al.** *Science,* 1994, vol. 264, 716-719
- **DE GROOT et al.** *Emerging Infectious Diseases,* 2001, vol. 7, 4
- **OJCIUS, D.M et al.** *Journal of Immunology,* 1993, vol. 151 (11), 6020-6026
- **OJCIUS, D.M et al.** *Biochemical and Biophysical Research Communications,* 1993, vol. 197 (3), 1216-1222
- **SETTE, A et al.** *Mol Immunol,* 1994, vol. 31 (11), 813-22
- **SOLHEIM, J.C et al.** *J. Immunol.,* 1993, vol. 151 (10), 5387-5397
- **BLUESTONE, J.A et al.** *Journal of Exp. Med.,* 1992, vol. 176 (6), 1757-61
- **OSTERGAARD PEDERSEN, L et al.** *Eur J Immunol,* 2001, vol. 31 (10), 2986-96
- **DEDIER, S et al.** *J Immunol Methods,* 2001, vol. 255 (1-2), 57-66
- **PRILLIMAN, K.R. et al.** *Immunogenetics,* 1997, vol. 45, 379-385
- **PRILLIMAN, K.R. et al.** *Immunogenetics,* 1998, vol. 48, 89-97
- **PRILLIMAN, K.R et al.** *Tissue Antigens,* 1999, vol. 54 (5), 450-60
- **PRILLIMAN, K.R et al.** *J Immunol,* 1999, vol. 162 (12), 7277-84
- **SMITH, E.S et al.** Lethality-based selection of recombinant genes in mammalian cells; application to Identifying tumor antigens. *Nat Med,* 2001, vol. 7 (8), 967-72
- **HUCZKO, E.L et al.** Characteristics of endogenous peptides eluted from the class I MHC molecule HLA-B7 determined by mass spectrometry and computer modeling. *J. Immunol.,* 1993, vol. 151, 2572-2587
- **SCHEIBENBOGEN, C et al.** Analysis of the T cell response to tumor and viral peptide antigens by an IFNg-ELISPOT assay. *Int. J. Cancer,* 1997, vol. 71, 932-936
- **RINALDO, C.R., JR. et al.** Anti-Human Immunodeficiency virus type 1 (HIV-1) CD8(+) T-lymphocyte reactivity during combination antiretroviral therapy In HIV-1-infected patients with advanced Immunodeficiency. *J Virol,* 2000, vol. 74 (9), 4127-38
- **ACHOUR, A et al.** *AIDS Res Hum Retroviruses,* 1994, vol. 10 (1), 19-25
- **CHIBA, M et al.** *CTL. Arch Virol,* 1999, vol. 144 (8), 1469-85
- **SMITH, E.S et al.** *Nat Med,* 2001, vol. 7 (8), 967-72
- **KHILKO SN et al.** Measuring Interactions of MHC class 1 molecules using surface plasmon resonance. *J Immunol Methods,* 1995, vol. 183 (1), 77-94
- **PARKER KC.** Peptide binding to HLA-A2 and HLA-B27 isolated from Escherichia coll. Reconstitution of HLA-A2 and HLA-B27 heavy chain/beta 2-microglobulin complexes requires specific peptides. *J Biol Chem.,* 1992, vol. 267 (8), 5451-9
- **PARKER KC et al.** Sequence motifs important for peptide binding to the human MHC class I molecule, HLA-A2. *J Immunol.,* 1992, vol. 149 (11), 3580-7